# EUROPEAN PATENT SPECIFICATION

(11) **EP 1 610 786 B1**
(45) Date of publication and mention of the grant of the patent: **20.06.2007**
(21) Application number: 04726514.5
(22) Date of filing: 08.04.2004
(51) Int. Cl.: A61K 31/4545, C07D 211/32, C07D 405/06, C07D 401/12, C07D 401/14, A61P 25/28

(54) **4-(4-(HETEROCYCLYLALKOXY PHENYL)-1-(HETEROCYCLYL-CARBONYL)PIPERIDINE DERIVATIVES AND RELATED COMPOUNDS AS HISTAMINE H3 ANTAGONISTS FOR THE TREATMENT OF NEUROLOGICAL DISEASES SUCH AS ALZHEIMER'S**
4-(4-(HETEROCYCLYLALKOXY PHENYL)-1-(HETEROCYCLYL-CARBONYL)PIPERIDIN DERIVATE UND VERWANDTE VERBINDUNGEN ALS HISTAMIN H3 ANTAGONSITEN ZUR BEHANDLUNG VON NEUROLOGISCHEN ERKRANKUNGEN WIE ALZHEIMER'S
DERIVES DE 4-(4-(HETEROCYCLYLALKOXY)PHENYL-1-(HETEROCYCLYL-CARBONYL)PIPERIDINE ET COMPOSES ASSOCIES SERVANT D'ANTAGONISTES D'HISTAMINE H3 DESTINES AU TRAITEMENT DE TROUBLES NEUROLOGIQUES

(30) Priority: 10.04.2003 GB 0308333
(43) Date of publication of application: 04.01.2006
(73) Proprietor: GLAXO GROUP LIMITED, Greenford, Middlesex UB6 ONN (GB)
(72) Inventor: BAMFORD, Mark James, GlaxoSmithKline, Harlow Essex CM19 5AW (GB); DEAN, David Kenneth, GlaxoSmithKline, Harlow Essex CM19 5AW (GB); WILSON, David Matthew, GlaxoSmithKline, Harlow Essex CM19 5AW (GB)
(74) Representative: Goddard, Carolyn Janice
(86) International application number: PCT/EP2004/003985
(87) International publication number: WO 2004/089373

(56) References cited:
- WO-A-02/12214
- WO-A-02/068388
- WO-A-02/076440
- DE-A- 4 407 139

## Description

The present invention relates to novel phenyl piperidinyl derivatives having pharmacological activity, processes for their preparation, to compositions containing them and to their use in the treatment of neurological and psychiatric disorders.

DE 4407139 (Dr Karl Thomae GmbH) describe a series of aminoalkyl-phenyl-azacycloalkanes which are claimed to be useful in the treatment of hyperlipidaemia, atherosclerosis, skin disorders, mycoses and in poultry feed for cholesterol-lean egg production. WO 02/76925 (Eli Lilly) describes a series of compounds which are claimed to be histamine H3 antagonists. WO 02/12214 (Ortho McNeil Pharmaceutical Inc) describes a series of substituted aryloxyalkylamines which are claimed to be histamine H3 antagonists.

The histamine H3 receptor is predominantly expressed in the mammalian central nervous system (CNS), with minimal expression in peripheral tissues except on some sympathetic nerves (Leurs et al., (1998), Trends Pharmacol. Sci. 19, 177-183). Activation of H3 receptors by selective agonists or histamine results in the inhibition of neurotransmitter release from a variety of different nerve populations, including histaminergic and cholinergic neurons (Schlicker et al., (1994), Fundam. Clin. Pharmacol. 8, 128-137). Additionally, *in vitro* and *in vivo* studies have shown that H3 antagonists can facilitate neurotransmitter release in brain areas such as the cerebral cortex and hippocampus, relevant to cognition (Onodera et al., (1998), In: The Histamine H3 receptor, ed Leurs and Timmerman, pp255-267, Elsevier Science B.V.). Moreover, a number of reports in the literature have demonstrated the cognitive enhancing properties of H3 antagonists (e.g. thioperamide, clobenpropit, ciproxifan and GT-2331) in rodent models including the five choice task, object recognition, elevated plus maze, acquisition of novel task and passive avoidance (Giovanni et al., (1999), Behav. Brain Res. 104, 147-155). These data suggest that novel H3 antagonists and/or inverse agonists such as the current series could be useful for the treatment of cognitive impairments in neurological diseases such as Alzheimer's disease and related neurodegenerative disorders.

The present invention provides, in a first aspect, a compound of formula (I) or a pharmaceutically acceptable salt thereof: 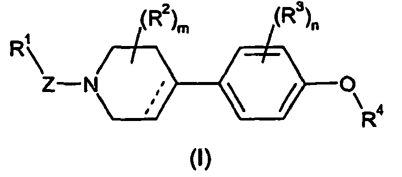 wherein:
R¹ represents -C₁₋₆ alkyl-O-C₁₋₆ alkyl, -C₃₋₈ cycloalkyl, aryl, heterocyclyl, heteroaryl, -C₁₋₆ alkyl-aryl, -C₁₋₆ alkyl-heteroaryl, -C₁₋₆ alkyl-heterocyclyl, -aryl-X-aryl, -aryl-X-heteroaryt, - aryl-X-heterocyclyl,- heteroaryl-X-aryl, -heteroaryl-X-heteroaryl, -heteroaryl-X-heterocyclyl, -heterocyclyl-X-aryl, -heterocyclyl-X-heteroaryl or -heterocyclyl-X-heterocyclyl,
   wherein said C₁₋₆ alkyl, C₃₋₈ cycloalkyl, aryl, heteroaryl and heterocyclyl groups of R¹ may be optionally substituted by one or more (eg. 1, 2 or 3) substituents which may be the same or different, and which are selected from the group consisting of halogen, hydroxy, cyano, nitro, oxo, haloC₁₋₆ alkyl, polyhaloC₁₋₆ alkyl, haloC₁₋₆ alkoxy, polyhaloC₁₋₆ alkoxy, C₁₋₆ alkyl, C₁₋₆ alkoxy, C₁₋₆ alkylthio, C₁₋₆ alkoxyC₁₋₆ alkyl, C₃₋₇ cycloalkylC₁₋₆ alkoxy, C₁₋₆ alkanoyl, C₁₋₆ alkoxycarbonyl, C₁₋₆ alkylsulfonyl, C₁₋₆ alkylsulfinyl, C₁₋₆ alkylsulfonyloxy, C₁₋₆ alkylsulfonylC₁₋₆ alkyl, C₁₋₆ alkylsulfonamidoC₁₋₆ alkyl, C₁₋₆ alkylamidoC₁₋₆ alkyl, arylsulfonyl, arylsulfonyloxy, aryloxy, arylsulfonamido, arylcarboxamido, aroyl, or a group NR¹⁵R¹⁶, -CONR¹⁵R¹⁶, -NR¹⁵COR¹⁶, -NR¹⁵SO₂R¹⁶ or -SO₂NR¹⁵R¹⁶, wherein R¹⁵ and R¹⁶ independently represent hydrogen or C₁₋₆ alkyl or together form a heterocyclic ring;
X represents a bond, O, CO, OCH₂, CH₂O or SO₂;
Z represents CO, CONR¹⁰ or SO₂;
R¹⁰ represents hydrogen, C₁₋₆ alkyl, -C₃₋₈ cycloalkyl, aryl, heterocyclyl, heteroaryl;
represents a single or a double bond;
m and n independently represent 0, 1 or 2;
R² represents hydrogen, C₁₋₆ alkyl or C₁₋₆ alkoxy;
R³ represents halogen, C₁₋₆ alkyl, hydroxy, C₁₋₆ alkoxy, cyano, amino, -COC₁₋₆ alkyl, - SO₂C₁₋₆ alkyl or trifluoromethyl;
R⁴ represents -(CH₂)_{q}-NR¹¹R¹² or a group of formula (i): 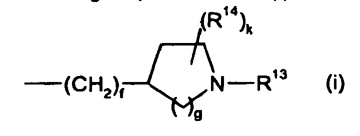 wherein q is 3 or 4;
-NR¹¹R¹² represents a heterocyclic group optionally substituted by one or more R¹⁷ groups;
R¹³ represents C₁₋₆ alkyl; C₃₋₈ cycloalkyl, -C₁₋₆ alkyl-C₁₋₆ alkoxy, -C₁₋₆ alkyl-C₃₋₈ cycloalkyl;
R¹⁴ and R¹⁷ independently represent halogen, C₁₋₆ alkyl, haloalkyl, OH or C₁₋₆ alkoxy;
fis 0 or 1;
g is 1 or 2
k is 0, 1 or 2
or a pharmaceutically acceptable salt thereof.

In one particular aspect of the present invention, there is provided a compound of formula (I) as defined above wherein:
said C₁₋₆ alkyl groups of R¹ may be optionally substituted by one or more (eg. 1, 2 or 3) substituents which may be the same or different, and which are selected from the group consisting of halogen, hydroxy, cyano, haloC₁₋₆ alkoxy, polyhaloC₁₋₆ alkoxy, C₃₋₇ cycloalkylC₁₋₆ alkoxy or C₁₋₆ alkanoyl; and
R³ represents halogen, C₁₋₆ alkyl, C₁₋₆ alkoxy, cyano, amino, -COC₁₋₆ alkyl, -SO₂C₁₋₆ alkyl or trifluoromethyl.

Alkyl groups, whether alone or as part of another group, may be straight chain or branched and the groups alkoxy and alkanoyl shall be interpreted similarly. The term 'halogen' is used herein to describe, unless otherwise stated, a group selected from fluorine, chlorine, bromine or iodine and the term 'polyhalo' is used herein to refer to a moiety containing more than one (eg. 2-5) of said halogen atoms.

The term "aryl" includes single and fused rings wherein at least one ring is aromatic, for example, phenyl, naphthyl and tetrahydronaphthalenyl.

The term "heterocyclyl" is intended to mean a 4-7 membered monocyclic saturated or partially unsaturated aliphatic ring or a 4-7 membered saturated or partially unsaturated aliphatic ring fused to a benzene ring containing 1 to 3 heteroatoms selected from oxygen, nitrogen or sulphur. Suitable examples of such monocyclic rings include pyrrolidinyl, azetidinyl, piperidinyl, piperazinyl, morpholinyl, tetrahydrofuranyl, tetrahydropyranyl, diazepanyl and azepanyl. Suitable examples of benzofused heterocyclic rings include indolinyl, isoindolinyl, 2,3,4,5-tetrahydro-1*H*-3-benzazepine or tetrahydroisoquinolinyl.

The term "heteroaryl" is intended to mean a 5-6 membered monocyclic aromatic or a fused 8-10 membered bicyclic aromatic ring containing 1 to 3 heteroatoms selected from oxygen, nitrogen and sulphur. Suitable examples of such monocyclic aromatic rings include thienyl, furyl, pyrrolyl, triazolyl, imidazolyl, oxazolyl, thiazolyl, oxadiazolyl, isothiazolyl, isoxazolyl, thiadiazolyl, pyrazolyl, pyrimidyl, pyridazinyl, pyrazinyl and pyridyl. Suitable examples of such fused aromatic rings include benzofused aromatic rings such as quinolinyl, isoquinolinyl, quinazolinyl, quinoxalinyl, cinnolinyl, naphthyridinyl, indolyl, indazolyl, pyrrolopyridinyl, benzofuranyl, benzothienyl, benzimidazolyl, benzoxazolyl, benzisoxazolyl, benzothiazolyl, benzisothiazolyl, benzoxadiazolyl, benzothiadiazolyl and the like.

Preferably, R¹ represents:
- aryl (eg. phenyl) optionally substituted by one or more (e.g. 1 or 2) halogen (eg. 4-fluorine), haloC₁₋₆ alkyl (eg. trifluoromethyl), cyano or SO₂Me groups;
- aryl-X-heterocyclyl (eg. -phenyl-CO-pyrrolidin-1-yl);
- heteroaryl (eg. pyridin-3-yl, pyridin-4-yl, pyrazinyl or quinoxalinyl) optionally substituted by one or more (e.g. 1 or 2) haloC₁₋₆ alkyl (eg. trifluoromethyl) or cyano groups;
- heterocyclyl (eg. tetrahydropyranyl, morpholinyl, piperidin-1-yl, pyrrofidin-1-yl, azetidin-1-yl or thiomorpholinyl) optionally substituted by one or more (e.g. 1 or 2) oxo groups; or
- C₁₋₆ alkyl-O-C₁₋₆ alkyl (eg. -(CH₂)₂OCH₃).

More preferably, R¹ represents -heterocyclyl (eg. tetrahydropyranyl) or -aryl (eg. phenyl) optionally substituted by a cyano group (eg. 4-cyanophenyl).
Also more preferably, R¹ represents -heteroaryl (eg. pyridin-3-yl) optionally substituted by a cyano (eg. 2-cyanopyridin-3-yl.) or haloC₁₋₆ alkyl (eg. 2-trifluoromethylpyridin-3-yl) group.

Most preferably, R¹ represents -aryl (eg. phenyl) optionally substituted by a cyano group (eg. 4-cyanophenyl).

Preferably, X and Z both represent CO.

Preferably, 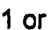 represents a single bond.

Preferably, m and n both represent 0.

When R⁴ represents -(CH₂)_{q}-NR¹¹R¹², preferably q represents 3 or 4 and -NR¹¹R¹² represents a heterocyclic group (eg. piperidinyl or pyrrolidinyl) optionally substituted by one or more (eg. 1 or 2) R¹⁷ groups.

When R⁴ represents -(CH₂)_{q}-NR¹¹R¹², more preferably q represents 3 and -NR¹¹R¹² represents a heterocyclic group (eg. piperidinyl or pyrrolidinyl) optionally substituted by one or more (eg. 1 or 2) R¹⁷ groups.

Preferably, R¹⁷ represents C₁₋₆ alkyl (eg. methyl).

When R⁴ represents a group of formula (i), preferably f and k both represent 0, g represents 2 and R¹³ represents C₁₋₆ alkyl (eg. i-propyl) or C₃₋₈ cycloalkyl (eg. cyclobutyl). Preferably, R⁴ represents -(CH₂)_{q}-NR¹¹R¹² wherein q represents 3 and -NR¹¹R¹² represents N-piperidinyl or N-pyrrolidinyl optionally substituted by 1 or 2 C₁₋₆ alkyl (eg. methyl) groups or R⁴ represents a group of formula (i) wherein f and k both represent 0, g represents 2 and R¹³ represents C₁₋₆ alkyl (eg. i-propyl) or C₃₋₈ cycloalkyl (eg. cyclobutyl).

More preferably, R⁴ represents a group of formula (i) wherein f and k both represent 0, g represents 2 and R¹³ represents C₁₋₆ alkyl (eg. i-propyl).

Preferred compounds according to the invention include examples E1-E56 as shown below, or a pharmaceutically acceptable salt thereof.

More preferred compounds according to the invention include:
5-{[4-(4-{[1-(1-Methylethyl)-4-piperidinyl] oxy}phenyl)-1-piperidinyl]carbonyl}-2-pyridinecarbonitrile; and
5-{[4-(4-{[1-(1-Methylethyl)-4-piperidinyl]oxy}phenyl)-1-piperidinyl]carbonyl}-2-(trifluoromethyl)pyridine;
or a pharmaceutically acceptable salt thereof.

A most preferred compound according to the invention is:
4-{[4-(4-{[1-(1-Methylethyl)-4-piperidinyl]oxy}phenyl)-1-piperidinyl] carbonyl} benzonitrile or a pharmaceutically acceptable salt thereof.

Compounds of formula (1) may form acid addition salts with acids, such as conventional pharmaceutically acceptable acids, for example maleic, hydrochloric, hydrobromic, phosphoric, acetic, fumaric, salicylic, sulfate, citric, lactic, mandelic, tartaric and methanesulphonic.

Certain compounds of formula (I) are capable of existing in stereoisomeric forms. It will be understood that the invention encompasses all geometric and optical isomers of these compounds and the mixtures thereof including racemates. Tautomers also form an aspect of the invention.

The present invention also provides a process for the preparation of a compound of formula (I) or a pharmaceutically acceptable salt thereof, which process comprises:
(a) preparing a compound of formula (I) wherein Z represents CO which comprises reacting a compound of formula (II) 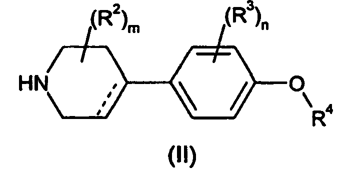 or an optionally activated or protected derivative thereof, wherein 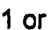 , R², R³, R⁴, m and n are as defined above, with a compound of formula R¹-CO-L¹, wherein R¹ is as defined above and L¹ represents a suitable leaving group such as a suitable halogen atom, or a hydroxyl group; or
(b) preparing a compound of formula (I) wherein Z represents SO₂ which comprises reacting a compound of formula (II) as defined above, with a compound of formula R¹-SO₂-L², wherein R¹ is as defined above and L² represents a suitable leaving group, such as a suitable halogen atom (eg. chlorine); or
(c) preparing a compound of formula (I) wherein Z represents CONH which comprises reacting a compound of formula (II) as defined above, with a compound of formula R¹-N=C=O, wherein R¹ is as defined above; or
(d) preparing a compound of formula (I) wherein Z represents CONR¹⁰ which comprises reacting a compound of formula (II) as defined above, with a compound of formula R¹R¹⁰N-L³, wherein R¹ and R¹⁰ are as defined above and L³ represents hydrogen or COCl; or
(e) deprotecting a compound of formula (I) or converting groups which are protected; and optionally thereafter
(f) interconversion to other compounds of formula (I).

When L¹ represents a halogen atom, process (a) typically comprises the use of a suitable base, such as triethylamine in an appropriate solvent such as dichloromethane. When L¹ represents a hydroxyl group, process (a) typically comprises the use of a coupling reagent, such as 1,3-dicyclohexylcarbodiimide and 1-hydroxybenzotriazole in an appropriate solvent such as dichloromethane.

Process (b) typically comprises the use of a base, such as triethylamine in an appropriate solvent such as dichloromethane.

Process (c) is typically conducted in a solvent such as dichloromethane.

When L³ represents hydrogen, process (d) typically comprises reacting the compound of formula (II) sequentially with phosgene in a suitable solvent such as toluene followed by the compound of formula R¹R¹⁰N-H in a suitable solvent such as dichloromethane.

When L³ represents COCl, process (d) typically comprises the use of a base, such as triethylamine in an appropriate solvent such as dichloromethane.

In process (e), examples of protecting groups and the means for their removal can be found in T. W. Greene 'Protective Groups in Organic Synthesis' (J. Wiley and Sons, 1991). Suitable amine protecting groups include sulphonyl (e.g. tosyl), acyl (e.g. acetyl, 2',2',2'-trichloroethoxycarbonyl, benzyloxycarbonyl or t-butoxycarbonyl) and arylalkyl (e.g. benzyl), which may be removed by hydrolysis (e.g. using an add such as hydrochloric acid) or reductively (e.g. hydrogenolysis of a benzyl group or reductive removal of a 2',2',2'-trichloroethoxycarbonyl group using zinc in acetic acid) as appropriate. Other suitable amine protecting groups include trifluoroacetyl (-COCF₃) which may be removed by base catalysed hydrolysis or a solid phase resin bound benzyl group, such as a Merrifield resin bound 2,6-dimethoxybenzyl group (Ellman linker), which may be removed by acid catalysed hydrolysis, for example with trifluoroacetic acid.

Process (f) may be performed using conventional interconversion procedures such as epimerisation, oxidation, reduction, alkylation, nucleophilic or electrophilic aromatic substitution, ester hydrolysis or amide bond formation. Examples of transition metal mediated coupling reactions useful as interconversion procedures include the following: Palladium catalysed coupling reactions between organic electrophiles, such as aryl halides, and organometallic reagents, for example boronic acids (Suzuki cross-coupling reactions); Palladium catalysed amination and amidation reactions between organic electrophiles, such as aryl halides, and nucleophiles, such as amines and amides; Copper catalysed amidation reactions between organic electrophiles (such as aryl halides) and nucleophiles such as amides; and Copper mediated coupling reactions between phenols and boronic acids.

Compounds of formula (II) wherein 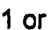 represents a single bond and R⁴ represents - (CH₂)_{q}-NR¹¹R¹² may be prepared in accordance with the following procedure: 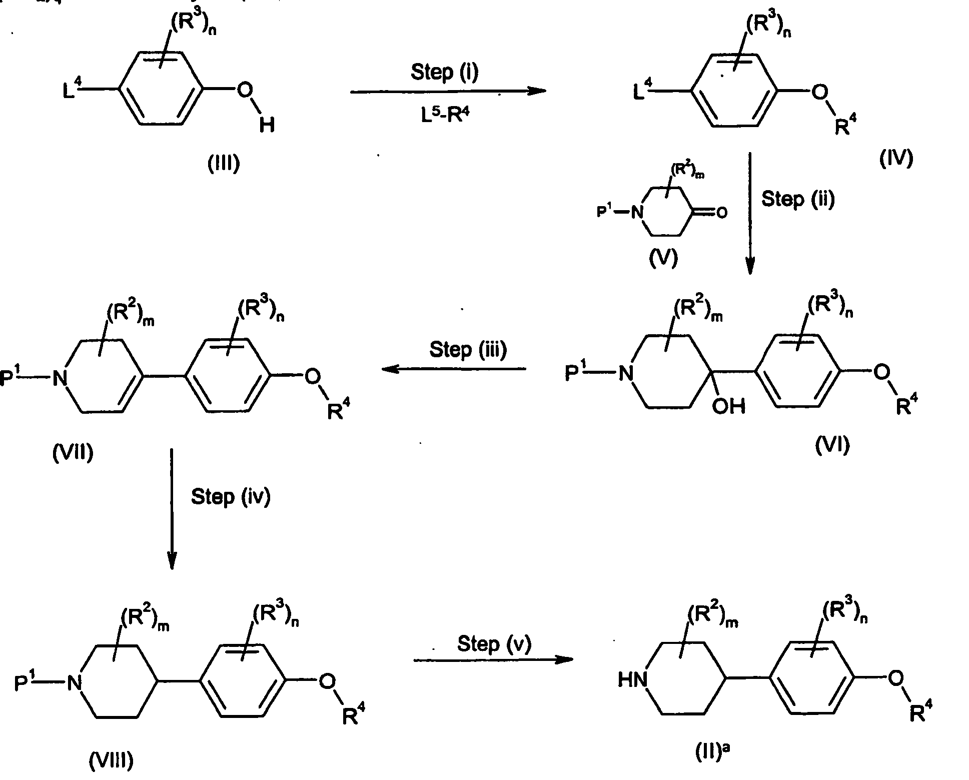 wherein R², R³, R⁴, m and n are as defined above, L⁴ represents a halogen atom (eg. iodine), L⁵ represents a suitable leaving group such as a suitable halogen atom (eg. bromine), or a hydroxyl group; and P¹ represents hydrogen or a suitable protecting group, such as t-butoxycarbonyl.

When L⁵ represents a halogen atom (eg. bromine or chlorine), step (i) may be performed using a suitable base, such as potassium carbonate in an appropriate solvent, such as 2-butanone, optionally in the presence of a transfer reagent, such as potassium iodide, at an appropriate temperature such as reflux.

When L⁵ represents a hydroxyl group, step (i) may be performed using a phosphine such as triphenylphosphine in a suitable solvent such as tetrahydrofuran, followed by addition of an azadicarboxylate such as diethylazaodicarboxylate at a suitable temperature such as room temperature.

Step (ii) may be performed by treating a compound of formula (IV) with an organo metallic reagent such as butyllithium under conditions suitable for metal-halogen exchange followed by treatment with a compound of formula (V).

Step (iii) may be performed under acidic conditions, for example, using trifluoroacetic acid in dichloromethane. Alternatively, steps (iii) and step (iv) may be performed together using a silane, such as triethylsilane, in the presence of an acid, for example, trifluoroacetic acid.

Step (iv) may be performed under transition metal catalysed hydrogenation conditions, for example, under a 50 psi pressure of hydrogen employing a suitable catalyst, such as palladium on charcoal, in a suitable solvent, such as ethanol.

Step (v) may be performed in accordance with the procedures outlined in process (e).

Compounds of formula (II) wherein 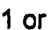 represents a double bond may be prepared in an identical manner to the procedure described above with the omission of step (iv).

Compounds of formula (II) wherein R⁴ represents a group of formula (i) may be prepared in an identical manner to the procedure described above except that the nitrogen atom of formula (i) may be optionally protected by a suitable protecting group such as Boc, prior to step (i). Step (i) will therefore be followed by a deprotection reaction as described in process (e) followed by introduction of an R¹³ group by, for example, reductive amination with acetone in the presence of a borohydride such as sodium triacetoxyborohydride and optionally an acid such as acetic acid in a suitable solvent such as dichloromethane, followed by steps (ii)-(v).

Compounds of formula (III) and (V) are either known or may be prepared in accordance with known procedures.

Compounds of formula (I) and their pharmaceutically acceptable salts have affinity for and are antagonists and/or inverse agonists of the histamine H3 receptor and are believed to be of potential use in the treatment of neurological diseases including Alzheimer's disease, dementia, age-related memory dysfunction, mild cognitive impairment, cognitive deficit, epilepsy, neuropathic pain, inflammatory pain, migraine, Parkinson's disease, multiple sclerosis, stroke and sleep disorders including narcolepsy; psychiatric disorders including schizophrenia (particularly cognitive deficit of schizophrenia), attention deficit hypereactivity disorder, depression and addiction; and other diseases including obesity, asthma, allergic rhinitis, nasal congestion, chronic obstructive pulmonary disease and gastro-intestinal disorders.

Thus the invention also provides a compound of formula (I) or a pharmaceutically acceptable salt thereof, for use as a therapeutic substance in the treatment or prophylaxis of the above disorders, in particular cognitive impairments in diseases such as Alzheimer's disease and related neurodegenerative disorders.

Thus, compounds of the invention may be used in a method of treatment or prophylaxis of the above disorders, In mammals including humane, which comprises administering to the sufferer a therapeutically effective amount of a compound of formula (I) or a pharmaceutically acceptable salt thereof.

In another aspect, the invention provides the use of a compound of formula (I) or a pharmaceutical acceptable salt thereof in the manufacture of a medicament for use in the treatment of the above disorders.

When used in therapy, the compounds of formula (I) are usually formulated in a standard pharmaceutical composition. Such compositions can be prepared using standard procedures.

Thus, the present invention further provides a pharmaceutical composition for use in the treatment of the above disorders which comprises the compound of formula (1) or a pharmaceutically acceptable salt thereof and a pharmaceutically acceptable carrier.

The present invention further provides a pharmaceutical composition which comprises the compound of formula (I) or a pharmaceutically acceptable salt thereof and a pharmaceutically acceptable carrier.

Compounds of formula (I) may be used in combination with other therapeutic agents, for example histamine H1 antagonists or medicaments claimed to be useful as either disease modifying or symptomatic treatments of Alzheimer's disease. Suitable examples of such other therapeutic agents may be agents known to modify cholinergic transmission such as 5-HTₑ antagonists, M1 muscarinic agonists, M2 muscarinic antagonists or acetylcholinesterase Inhibitors. When the compounds are used In combination with other therapeutic agents, the compounds may be administered either sequentially or simultaneously by any convenient route.

The invention thus provides, in a further aspect, a combination comprising a compound of formula (I) or a pharmaceutically acceptable derivative thereof together with a further therapeutic agent or agents.

The combinations referred to above may conveniently be presented for use in the form of a pharmaceutical formulation and thus pharmaceutical formulations comprising a combination as defined above together with a pharmaceutically acceptable carrier or excipient comprise a further aspect of the invention. The individual components of such combinations may be administered either sequentially or simultaneously in separate or combined pharmaceutical formulations.

When a compound of formula (I) or a pharmaceutically acceptable derivative thereof is used in combination with a second therapeutic agent active against the same disease state the dose of each compound may differ from that when the compound is used alone. Appropriate doses will be readily appreciated by those skilled in the art.

A pharmaceutical composition of the invention, which may be prepared by admixture, suitably at ambient temperature and atmospheric pressure, is usually adapted for oral, parenteral or rectal administration and, as such, may be in the form of tablets, capsules, oral liquid preparations, powders, granules, lozenges, reconstitutable powders, injectable or infusible solutions or suspensions or suppositories. Orally administrable compositions are generally preferred.

Tablets and capsules for oral administration may be in unit dose form, and may contain conventional excipients, such as binding agents, fillers, tabletting lubricants, disintegrants and acceptable wetting agents. The tablets may be coated according to methods well known in normal pharmaceutical practice.

Oral liquid preparations may be in the form of, for example, aqueous or oily suspension, solutions, emulsions, syrups or elixirs, or may be in the form of a dry product for reconstitution with water or other suitable vehicle before use. Such liquid preparations may contain conventional additives such as suspending agents, emulsifying agents, non-aqueous vehicles (which may include edible oils), preservatives, and, if desired, conventional flavourings or colorants.

For parenteral administration, fluid unit dosage forms are prepared utilising a compound of the invention or pharmaceutically acceptable salt thereof and a sterile vehicle. The compound, depending on the vehicle and concentration used, can be either suspended or dissolved in the vehicle. In preparing solutions, the compound can be dissolved for injection and filter sterilised before filling into a suitable vial or ampoule and sealing. Advantageously, adjuvants such as a local anaesthetic, preservatives and buffering agents are dissolved in the vehicle. To enhance the stability, the composition can be frozen after filling into the vial and the water removed under vacuum. Parenteral suspensions are prepared in substantially the same manner, except that the compound is suspended in the vehicle instead of being dissolved, and sterilisation cannot be accomplished by filtration. The compound can be sterilised by exposure to ethylene oxide before suspension in a sterile vehicle. Advantageously, a surfactant or wetting agent is included in the composition to facilitate uniform distribution of the compound.

The composition may contain from 0.1 % to 99% by weight, preferably from 10 to 60% by weight, of the active material, depending on the method of administration. The dose of the compound used in the treatment of the aforementioned disorders will vary in the usual way with the seriousness of the disorders, the weight of the sufferer, and other similar factors. However, as a general guide suitable unit doses may be 0.05 to 1000 mg, more suitably 1.0 to 200 mg, and such unit doses may be administered more than once a day, for example two or three a day. Such therapy may extend for a number of weeks or months.

The following Descriptions and Examples illustrate the preparation of compounds of the invention.

### Description 1

### 1-(Phenylmethyl)-4-(4-{[3-(1-piperidinyl)propyl]oxy}phenyl)-4-piperidinol (D1)

A solution of 1-(3-[(4-iodophenyl)oxy]propyl)piperidine (WO 02/12214) (1.0g, 2.9mmol) in THF (5ml) at -70°C was treated with n-butyl lithium (1.6M in hexanes, 2ml, 3.2mmol). After stirring at -70°C for 30 minutes 1-(phenylmethyl)-4-piperidinone (548mg, 2.9mmol) was added dropwise and the mixture stirred for 1 hour. Saturated ammonium chloride solution was added and the mixture was extracted with ethyl acetate. The organic phase was washed with water and brine, dried over anhydrous sodium sulfate and evaporated *in vacuo* to yield a residue which was purified using silica gel chromatography eluting with a mixture of 0.880 ammonia:ethanol:dichloromethane (0.5:4.5:95) to afford the title compound (550mg, 47%); MS (ES+), m/e 409 [M+H]⁺.

### Description 2

### 1-(Phenylmethyl)-4-(4-{[3-(1-piperidinyl)propyl]oxy}phenyl)-1,2,3,6-tetrahydro pyridine (D2)

To a solution of 1-(phenylmethyl)-4-(4-{[3-(1-piperidinyl)propyl]oxy}phenyl)-4-piperidinol (D1) (450mg, 1.1 mmol) in dichloromethane (5ml) was added trifluoroacetic acid (0.68ml, 8.8mmol) and powdered 4A molecular sieves. After stirring at room temperature for 2 hours the suspension was filtered and the filtrate evaporated *in vacuo.* The residue was dissolved in dichloromethane and stirred with aqueous sodium hydroxide solution for 10 minutes. The organic phase was separated, washed with water and brine, dried over anhydrous sodium sulfate and evaporated *in-vacuo* to afford the title compound (365mg, 85%); MS (ES+), m/e 391 [M+H]⁺..

### Description 3

### 1-(3-{[4-(4-Piperidinyl)phenyl]oxy}propyl)piperidine (D3)

A solution of 1-(phenylmethyl)-4-(4-{[3-(1-piperidinyl)propyl]oxy}phenyl)-1,2,3,6-tetrahydro pyridine (D2) (450mg, 1.15mmol) in methanol (40ml) containing palladium on charcoal (10% paste, 200mg) was hydrogenated at 50 p.s.i. at room temperature for 18 hours. The mixture was filtered through filter aid and the filtrate evaporated *in vacuo* to afford the title compound (330mg, 95%); MS (ES+), m/e 303 [M+H]⁺.

### Description 4

### 1, 1-Dimethylethyl 4-[(4-iodophenyl)oxy]-1-piperidinecarboxylate (D4)

Di-tert-butyl azodicarboxylate (57g; 250mmol) was added portionwise to a stirring mixture of 4-iodophenol (50g; 230mmol), triphenyl phosphine (65.6g; 250mmol) and 1,1-dimethylethyl 4-hydroxy-l-piperidinecarboxylate (50g; 250mmol) in dry tetrahydrofuran and cooled to 0°C. The resulting mixture was stirred at room temperature for 3 days. The solvent was removed by filtration and the residue purified by column chromatography on silica eluting with a mixture of n- hexane and ethyl acetate (9:1). Fractions containing the product were combined and evaporated to afford the title compound as a white crystalline solid (63.4g, 63%), MS (ES+), m/e 404 [M+H]⁺.

### Description 5

### Phenylmethyl 4-[4-[(1-{[1(1,1-dimethylethyl)oxy]carbonyl}-4-piperidinyl)oxy]phenyl}-4-hydroxy-1-piperidinecarboxylate (D5)

A solution of 1,1-dimethylethyl 4-[(4-iodophenyl)oxy]-l-piperldinecarboxylate (10g; 24.8 mM) (D4) in THF (100 ml) at -70°C was treated with n-butyl lithium (1.6M in hexanes, 23 ml, 36.8 mmol). After stirring at -70°C for 30 minutes a solution of 4-oxo-piperidine-1-carboxylic acid benzyl ester (8.7 g, 36.8 mmol) in THF was added dropwise and the mixture stirred for 18 hours. Saturated ammonium chloride solution was added and the mixture was extracted with ethyl acetate. The organic phase was washed with water and brine, dried over anhydrous sodium sulfate and evaporated *in vacuo* to yield a residue which was purified using silica gel chromatography eluting with a mixture of hexane and ethyl acetate (1:1) to afford the title compound (7.38 g, 56%); MS (ES+), m/e 511 [M+H]⁺.

### Description 6

### Phenylmethyl 4-[4-(4-piperidinyloxy)phenyl]-3,6-dihydro-1(2H)-pyridinecarboxylate (D6)

Trifluoroacetic acid (12 ml) was added to a stirring solution of phenylmethyl 4-{4-[(1-{[(1,1-dimethylethyl)oxy]carbonyl}-4-piperidinyl)oxy]phenyl}-4-hydroxy-1-piperidinecarboxylate (7.38g; 14.5mmol) (D5) in dichloromethane (12ml) and the mixture stirred for 60 minutes. The solvent was removed by evaporation and the residue filtered through a SCX column eluting with methanol followed by 10% 0.880 ammonia solution in methanol to elute the product (4.9g; 87%). MS (ES+), m/e 393 [M+H]⁺.

### Description 7

### Phenylmethyl 4-(4-{[1-(1-methylethyl)-4-piperidinyl]oxy}phenyl)-3,6-dihydro-1(2H)-pyridinecarboxylate (D7)

Sodium triacetoxyborohydride (5.3g; 25.2mmol) was added portion-wise to a stirring mixture of phenylmethyl 4-[4-(4-piperidinyloxy)phenyl]-3,6-dihydro-1 (2H)-pyridinecarboxylate (4.94g; 12.6mmol) (D6), acetone (5ml; 63mmol) and glacial acetic acid (1ml) in dichloromethane (60ml). The mixture was stirred at room temperature for 18 hours. The mixture was stirred with aqueous sodium hydroxide solution for 10 minutes. The organic phase was separated, washed with water and brine, dried over anhydrous sodium sulfate and evaporated *in vacuo* to afford the title compound (5.11g, 93%); MS (ES+), m/e 435 [M+H]⁺.

### Description 8

### 1-(1-Methylethyl)-4-{[4-(4-piperidinyl)phenyl]oxy}piperidine (D8)

A solution of phenylmethyl 4-(4-{[1-(1-methylethyl)-4-piperidinyl]oxy}phenyl)-3,6-dihydro-1(2H)-pyridinecarboxylate (5.11 g, 11.8mmol) (D7) in ethanol (75ml) containing palladium on charcoal (10% paste, 1g) was hydrogenated at 50 p.s.i. at room temperature for 18 hours. The mixture was filtered through filter aid and the filtrate evaporated *in vacuo* to afford the title compound (3.51 g, 98%); MS (ES+), m/e 303 [M+H]⁺.

### Description 9

### Phenylmethyl 4-{4-[(1-cyclobutyl-4-piperidinyl)oxy]phenyl}-3,6-dihydro-1(2H)-pyridinecarboxylate (D9)

Sodium triacetoxyborohydride (2.13g; 10.05mmol) was added portion-wise to a stirring mixture of phenylmethyl 4-[4-(4-piperidinyloxy)phenyl]-3,6-dihydro-1(2*H*)-pyridinecarboxylate (1.97g; 5.03mmol) (D6), cyclobutanone (0.8ml; 10.05mmol) and 4 molecular sieves in dichloromethane (50ml). The mixture was stirred at room temperature for 4 hours. The mixture was stirred with aqueous sodium hydroxide solution for 10 minutes. The organic phase was separated, washed with water and brine, dried over anhydrous sodium sulfate and evaporated *in vacuo* to afford the title compound (1.95g, 87%); MS (ES+), m/e 447 [M+H]⁺.

### Description 10

### 1-Cyclobutyl-4-{[4-(4-piperidinyl)phenyl]oxy}piperidine (D10)

A solution of phenylmethyl 4-{4-[(1-cyclobutyl-4-piperidinyl)oxy]phenyl}-3,6-dihydro-1(*2H*)-pyridinecarboxylate (1.95g, 4.37mmol) (D9) in ethanol (40ml) containing palladium on charcoal (10% paste, 0.4g) was hydrogenated at 50 p.s.i. at room temperature for 18 hours. The mixture was filtered through filter aid and the filtrate evaporated *in vacuo* to afford the title compound (1.34g, 96%); MS (ES+), m/e 315 [M+H]⁺.

### Description 11

### 1,1-Dimethylethyl 4-hydroxy-4-(4-{[3-(1-piperidinyl)propyl]oxy}phenyl)-1-plperidinecarboxylate (D11)

A solution of 1-{3-[(4-iodophenyl)oxy]propyl}piperidine (WO 02/12214) (10g; 29 mmol) in THF (50 ml) at-70°C was treated with *n*-butyl lithium (1.6M in hexanes, 21.8ml, 34.8mmol). After stirring at -70°C for 30 minutes a solution of 1,1-dimethylethyl 4-oxo-1-piperidinecarboxylate (6.36g, 31.9mmol) in THF (15 ml) was added dropwise and the mixture stirred for 2 hours. Saturated ammonium chloride solution was added and the mixture was extracted with ethyl acetate. The organic phase was washed with water and brine, dried over anhydrous sodium sulfate and evaporated *in vacuo* to yield a residue which was purified using silica gel chromatography eluting with a mixture of 1-9-90 0.88 aqueous ammonia solution-methanol-DCM to afford the title compound (5.2 g, 45%); MS (ES+), m/e 419 [M+H]⁺.

### Description 12

### 4-(4-{[3-(1 -Piperidinyl)propyl]oxy}phenyl)-1,2,3,6-tetrahydropyridine (D12)

Trifluoroacetic acid (6ml) was added to a stirring solution of 1,1-dimethylethyl 4-hydroxy-4-(4-{[3-(1-piperidinyl)propyl]oxy}phenyl)-piperidinecarboxylate (3.9g; 9.3mmol) (D11) in dichloromethane (6ml) and the mixture stirred for 60 minutes. The solvent was removed by evaporation and the residue filtered through a SCX column eluting with methanol followed by 10% 0.880 ammonia solution in methanol to elute the product (2.1g; 77%), MS (ES+), m/e 301 [M+H]⁺.

### Description 13

### 4-[(4-Iodophenyl)oxy]piperidine (D13)

Trifluoroacetic acid (10ml) was added to a stirring solution of 1,1-dimethylethyl 4-[(4-iodophenyl)oxy]-1-piperidinecarboxylate (10g; 24.8mmol) (D4) in dichloromethane (10ml) and the mixture stirred for 60 minutes. The solvent was removed by evaporation and the residue basified using 2M sodium hydroxide solution. This mixture was extracted with ethyl acetate (x3) and the extracts combined. These were dried using sodium sulfate and the solvent removed by evaporation to give the product (6.63g, 88%), MS (ES+), m/e 304 [M+H]⁺.

### Description 14

### 4-[(4-lodophenyl)oxy]-1-(1-methylethyl)piperidine (D14)

Sodium triacetoxyborohydride (9.3g; 44mmol) was added portion-wise to a stirring mixture of 4-[(4-iodophenyl)oxy]piperidine (6.6g; 21 mmol) (D13) and acetone (8ml; 63mmol) in dichloromethane (60ml) and glacial acetic acid (1ml). The mixture was stirred at room temperature for 18 hours. The mixture was stirred with aqueous sodium hydroxide solution for 10 minutes. The organic phase was separated, washed with water and brine, dried over anhydrous sodium sulfate and evaporated *in vacuo* to afford the title compound (7.2g, 96%); MS (ES+), m/e 346 [M+H]⁺.

### Description 15

### 1,1-Dimethylethyl 4-hydroxy-4-(4-{[1-(1-methylethyl)-4-piperidinyl]oxy}phenyl)-1-piperidinecarboxylate (D15)

A solution of 4-[(4-lodophenyl)oxy]-1-(1-methylethyl)piperidine (7.2g; 20.9mmol) (D14) in THF (100ml) at -70°C was treated with n-butyl lithium (2.5M in hexanes, 12.6ml, 31.4mmol). After stirring at -70°C for 30 minutes a solution of 1,1-dimethylethyl 4-oxo-1-piperidinecarboxylate (6.25g, 31.4mmol) in THF (25 ml) was added dropwise and the mixture stirred for 18 hours. Saturated ammonium chloride solution was added and the mixture was extracted with ethyl acetate. The organic phase was washed with water and brine, dried over anhydrous sodium sulfate and evaporated *in vacuo* to yield a residue which was purified using silica gel chromatography eluting with a mixture of 1-9-90 0.88 aqueous ammonia solution-methanol-DCM to afford the title compound (2.9 g, 33%); MS (ES+), m/e 419 [M+H]⁺.

### Description 16

### 4-(4-{[1-(1-Methylethyl)-4-piperidinyl]oxy}phenyl)-1,2,3,6-tetrahydropyridine (D16)

Trifluoroacetic acid (6ml) was added to a stirring solution of 1,1-dimethylethyl 4-hydroxy-4-(4-{[1-(1-methylethyl)-4-piperidinyl]oxy}phenyl)-1-piperidinecarboxylate (2.9g; 6.9mmol) (D15) in dichloromethane (6ml) and the mixture stirred for 30 minutes. The solvent was removed by evaporation and the residue filtered through a SCX column eluting with methanol followed by 10% 0.880 ammonia solution in methanol to elute the product (2.0g; 97%). MS (ES+), m/e 301 [M+H]⁺.

### Description 17

### 1-[(3-Chloropropyl)oxy]-4-iodobenzene (D17)

A mixture of 4-iodophenol (10g; 45.5 mmol), 1-bromo-3-chloro propane (9g; 56.8 mmol) and potassium carbonate (12.6g; 91 mmol) in 2-butanone (150ml) was heated at reflux for 3 days. The solid was removed by filtration and the solvent removed by evaporation. The residue was purified by column chromatography on silica eluting with 20-1 n-pentane - ethyl acetate to afford the product as a colourless oil (13.0g; 96%), MS (ES+), m/e 296 & 298 [M+H]⁺.

### Description 18

### (2R)-1-{3-[(4-Iodophenyl)oxy]propyl}-2-methylpyrrolidine (D18)

A mixture of 1-[(3-chloropropyl)oxy]-4-iodobenzene (13g; 43.8 mmol) (D17), (2R)-2-methylpyrrolidine (2.63g; 15.8 mmol), potassium carbonate (6.6g; 43.8 mmol) and potassium iodide (7.9g; 43.8 mmol) in acetonitrile (100ml) was heated at reflux for 18 hours. The solid was removed by filtration and the solvent removed by evaporation. The residue was poured into water and extracted with ethyl acetate. The extracts were combined, dried with sodium sulfate and evaporated. This residue was purified by column chromatography on silica eluting with 20-1 dichloromethane - 2M ammonia in methanol to afford the product as a yellow solid (2.41g; 44%), MS (ES+), m/e 346 [M+H]⁺.

### Description 19

### 1,1-Dimethylethyl 4-hydroxy-4-[4-({3-[(2R)-2-methyl-1-pyrrolidinyl]propyl}oxy) phenyl-1-piperidinecarboxylate (D19)

A solution of (2*R*)-1-{3-[(4-iodophenyl)oxy]propyl}-2-methylpyrrolidine (2.4g; 6.95mmol) (D18) in THF (40ml) at-70°C was treated with n-butyl lithium (2.5M in hexanes, 3.5ml, 8.69mmol). After stirring at -70°C for 30 minutes a solution of 1,1-dimethylethyl 4-oxo-1-piperidinecarboxylate (1.73g, 8.69mmol) in THF (10 ml) was added dropwise and the mixture stirred at room temperature for 18 hours. Saturated ammonium chloride solution was added and the mixture was extracted with ethyl acetate. The organic phase was washed with water and brine, dried over anhydrous sodium sulfate and evaporated *in vacuo* to yield a residue which was purified using silica gel chromatography eluting with 20-1 dichloromethane - 2M ammonia in methanol to afford the title compound (0.75g, 26%); MS (ES+), m/e 419 [M+H]⁺.

### Description 20

### 4-[4-({3-[(2R)-2-Methyl-1-pyrrolidinyl]propyl}oxy)phenyl]-1,2,3,6-tetrahydropyridine (D20)

A solution of 1,1-dimethylethyl 4-hydroxy-4-[4-({3-[(2*R*)-2-methyl-1-pyrrolidinyl] propyl}oxy) phenyl-1-piperidinecarboxylate (0.75g; 1.8mmol) (D19) in a mixture of dichloromethane (10ml) and trifluoroacetic acid (10ml) was stirred at room temperature for 2 hours. The solvent was removed by evaporation to give the product as its di-trifluoroacetate salt (1.22g), MS (ES+), m/e 301 [M+H]⁺.

### Description 21

### 4-[4-({3-[(2R)-2-Methyl-1-pyrrolidinyl]propyl}oxy)phenyl]piperidine (D21)

A solution of 4-[4-({3-[(2*R*)-2-methyl-1-pyrrolidinyl]propyl}oxy)phenyl]-1,2,3,6-tetrahydropyridine (1.95g, 4.49mmol) (D20) in ethanol (20ml) containing palladium on charcoal (10% paste, 0.4g) was hydrogenated at 50 p.s.i. at room temperature for 18 hours. The mixture was filtered through filter aid and the filtrate evaporated *in vacuo* to afford the title compound as its di-trifluoroacetate salt (1.05g); MS (ES+), m/e 303 [M+H]⁺.

### Description 22

### 1,1-Dimethylethyl 4-hydroxy-4-{4-[(phenylmethyl)oxy]phenyl}-1-piperidine carboxylate (D22)

A solution of 1-iodo-4-[(phenylmethyl)oxy]benzene (25g; 80.6 mmol) in THF (300 ml) at-70°C was treated with n-butyl lithium (2.5M in hexanes, 40.3 ml, 0.10 mol). After stirring at -70°C for 30 minutes a solution of 1,1-dimethylethyl4-0xo-1-piperidine carboxylate (20.1g, 0.10 mol) in THF (150 ml) was added dropwise and the mixture stirred at room temperature for 18 hours. Saturated ammonium chloride solution was added and the mixture was extracted with ethyl acetate. The organic phase was washed with water and brine, dried over anhydrous sodium sulfate and evaporated *in vacuo* to yield a residue which was purified using silica gel chromatography eluting with 4-1 hexane - ethyl acetate to afford the title compound (15.9 g, 51%); NMR (CDCl₃), δ1.48 (s, 9H), 1.73 (m, 2H). 1.95 (m, 2H), 3.25 (m, 2H), 4.00 (m, 2H), 5.07 (s, 2H), 6.97 (m, 2H), 7.37-7.44 (m, 7H)..

### Description 23

### 4-{4-[(Phenylmethyl)oxy]phenyl}-1,2,3,6-tetrahydropyridine (D23)

Trifluoroacetic acid (35 ml) was added drop wise to a stirring solution of 1,1-dimethylethyl 4-hydroxy-4-{4-[(phenylmethyl)oxy]phenyl}-1-piperidine carboxylate (16.3g; 42.5 mmol) (D22) in dichloromethane (35 ml). After 2 hours the solvent was removed by evaporation and the residue poured into 2M sodium hydroxide solution. The mixture was filtered, the solid washed with water and dried (7.2g; 64%), MS (ES+), m/e 266 [M+H]⁺

### Description 24

### 4-{4-[(Phenylmethyl)oxy]phenyl}-1-(tetrahydro-2H-pyran-4-ylcarbonyl)-1,2,3,6-tetrahydropyridine (D24)

Ethanedioyl dichloride (5.1 ml; 58 mmol) was added to a solution of tetrahydro-2*H*-pyran-4-carboxylic acid (7.4g; 57 mmol) in dichloromethane (50 ml) and dimethyl formamide (0.2 ml). After stirring at room temperature for 3 hours the solvent was removed by evaporation and the residue dissolved in dichloromethane (30 ml). This solution was added dropwise to a mixture of 4-{4-[(phenylmethyl)oxy]phenyl}-1,2,3,6-tetrahydropyridine (14.4g; 54.2 mmol) (D23) and triethylamine (8.3 ml; 59.6 mmol) in dichloromethane (100 ml). After 1 hour this mixture was washed with 1 M hydrochloric acid, water, 1M sodium carbonate solution and brine, dried with sodium sulfate and evaporated. The residue was purified by column chromatography on silica eluting with 98-2 dichloromethane - methanol to give a white solid (6.87g; 34%), MS (ES+), m/e 378 [M+H]⁺

### Description 25

### 4-[1-(Tetrahydro-2H-pyran-4-ylcarbonyl)-4-piperidinyl]phenol (D25)

A solution of 4-{4-[(phenylmethyl)oxy]phenyl}-1-(tetrahydro-*2H*-pyran-4-ylcarbonyl)-1,2,3,6-tetrahydropyridine (13.6g; 36.1 mmol) (D24) in tetrahydrofuran (500 ml) was hydrogenated at 50 psi and room temperature over 10% palladium on carbon. After 18 hours the catalyst was removed by filtration and the filtrate evaporated to give colourless crystals (10.1g; 96%), MS (ES+), m/e 290 [M+H]⁺.

### Description 26

### 4-{4-[(3-Chloropropyl)oxy]phenyl}-1-(tetrahydro-2H-pyran-4-ylcarbonyl)piperidine (D26)

A mixture of 4-[1-(tetrahydro-2H-pyran-4-ylcarbonyl)-4-piperidinyl]phenol (2.57g; 8.9 mmol) (D25), 1-bromo-3-chloropropane (1.1 ml; 10.7 mmol) and potassium carbonate (2.5g; 17.8 mmol) in 2-butanone (100ml) was heated at reflux for 18 hours. The solid was removed by filtration and the solvent removed by evaporation. The residue was poured into water and extracted with ethyl acetate. The extracts were combined, dried with sodium sulfate and evaporated. This residue was purified by column chromatography on silica eluting with 1-1 hexane - ethylacetate to afford the product as a colourless oil (2.54g; 78%), MS (ES+), m/e 366 [M+H]⁺.

### Description 27

### 3-({4-[1-(Tetrahydro-2H-pyran-4-ylcarbonyl)-4-piperidinyl]phenyl}oxy)-1-propanol (D27)

A mixture of 4-[1-(tetrahydro-2*H*-pyran-4-ylcarbonyl)-4-piperidinyl]phenol (10.1g; 34.7 mmol) (D25), 3-chloro-1-propanol (4.3 ml; 43.4 mmol) and potassium carbonate (9.6g; 69.4 mmol) in 2-butanone (200ml) was heated at reflux for 18 hours. The solid was removed by filtration and the solvent removed by evaporation. The residue was poured into water and extracted with ethyl acetate. The extracts were combined, dried with sodium sulfate and evaporated. This residue was purified by column chromatography on silica eluting with 1-1 hexane - ethylacetate to afford the product as a colourless solid (9.32g; 77%), MS (ES+), m/e 348 [M+H]⁺.

### Description 28

### 3-({4-[1-(Tetrahydro-2H-pyran-4-ylcarbonyl)-4-piperidinyl]phenyl}oxy)propyl methanesulfonate (D28)

A solution of methane sulphonyl chloride (0.22 ml; 2.9 mmol) in dichloromethane (5 ml) was added to a mixture of 3-({4-[1-(tetrahydro-2*H*-pyran-4-ylcarbonyl)-4-piperidinyl]phenyl}oxy)-1-propanol (0.5 g; 1.44 mmol) (D27) and triethylamine (0.6 ml; 4.3 mmol) in dichloromethane (15 ml). After 1 hour the mixture was washed with water, dried with sodium sulfate and evaporated to give a yellow solid (0.61 g; 99%), NMR (CDCl₃), δ 1.55-1.65 (6H, m), 1.90-1.98 (4H, m), 2.22 (2H, m), 2.62-2.81 (3H, m), 2.99 (3H, s), 3.14 (H, m), 3.46 (2H, t), 4.02-4.09 (5H, m), 4.80 (H, m), 6.85 (2H, m), 7.10 (2H, m)

### Example 1

### 4-(4-{[3-(1-Piperidinyl)propyl]oxy}phenyl)-1-(tetrahydro-2H-pyran-4-ylcarbonyl)piperidine (E1)

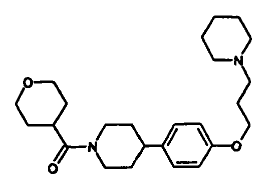

A mixture of tetrahydro-pyran-4-carboxylic acid (130mg, 1mmol), 1-hydroxybenzotriazole hydrate (135mg, 1mmol) and N-cyclohexylcarbodiimide-N'-methyl polystyrene (550mg, 1mmol, resin loading 1.8mmol/g) in dichloromethane (8ml) was stirred at room temperature for 15 minutes. A solution 1-(3-{[4-(4-piperidinyl)phenyl]oxy}propyl) piperidine (D3) (151 mg, 0.5mmol) in dichloromethane (5ml) was added and the mixture stirred at room temperature for 24 hours. The mixture was filtered through a SCX column eluting with methanol followed by 10% 0.880 ammonia solution in methanol to elute the product. The residue was purified by silica gel chromatography eluting with a 1:9:90 mixture of 0.880 ammonia solution:methanol:dichloromethane to afford the title compound (127mg, 63%); MS(AP+) m/e 415 [M+H]⁺.

### Example 1 (Alternative Preparation)

### 4-(4-{[3-(1-Piperidinyl)propyl]oxy}phenyl)-1-(tetrahydro-2H-pyran-4-ylcarbonyl)piperidine (E1)

A mixture of 4-{4-[(3-chloropropyl)oxy]phenyl}-1-(tetrahydro-2*H*-pyran-4-ylcarbonyl)piperidine (14.33g; 39.2 mmol) (D26), piperidine (7.5 ml; 78.4 mmol), potassium carbonate (10.8g; 78.4 mmol) and potassium iodide (13.0g; 78.4 mmol) in 2-butanone (120ml) was heated at reflux for 18 hours. The solid was removed by filtration and the solvent removed by evaporation. This residue was purified by column chromatography on silica eluting with 5-95 2M ammonia in methanol - dichloromethane to afford the product as a colourless solid (14.7g; 90%), MS (ES+), m/e 415 [M+H]⁺.

### Examples 2-7

Examples 2-7 (E2-E7) were prepared from 1-(3-{[4-(4-piperidinyl)phenyl]oxy}propyl)piperidine (D3) using an analogous method to that described in Example 1 (E1) by substituting tetrahydro-pyran-4-carboxylic acid for the appropriate acid indicated in the table.

| **Example** | **Acid** | **Mass Spectrum** |
|---|---|---|
| 4-{[4-(4-{[3-(1-Piperidinyl)propyl]oxy}phenyl)-1-piperidinyl]carbonyl}benzonitrile (E2) | 4-cyanobenzoic acid | MS (ES+), m/e 432 [M+H]⁺. |
| 4-{[4-(4-{[3-(1-Piperidinyl)propyl]oxy}phenyl)-1-piperidinyl]carbonyl}pyridine (E3) | Isonicotinic acid | MS (ES+), m/e 408 [M+H]⁺. |
| 4-(4-{[3-(1-Piperidinyl)propyl]oxy}phenyl)- 1-{[4-(1-pyrrolidinylcarbonyl)phenyl] carbonyl} piperidine (E4) | 4-(1-pyrrolidin-1-yl-methanoyl)-benzoic add (J. Med. Chem. 46(10), 1845, 2003) | MS (ES+), m/e 504 [M+H]⁺. |
| 1-{[4-(Methylsulfonyl)phenyl]carbonyl}-4-(4-{[3-(1-piperidinyl) propyl] oxy} phenyl) piperidine (E5) | 4-methanesulfonyl-benzoic acid | MS (ES+), m/e 485 [M+H]⁺. |
| 1-[(4-Fluorophenyl)carbonyl]-4-(4-{[3-(1-piperidinyl)propyl]oxy}phenyl)piperidine (E6) | 4-fluorobenzoic acid | MS (ES+), m/e 425 [M+H]⁺. |
| 3-{[4-(4-{[3-(1-Piperidinyl)propyl]oxy}phenyl)-1-piperidinyl]carbonyl}pyridine (E7) | Nicotinic acid | MS (ES+), m/e 408 [M+H]⁺. |

### Example 8

### 4-{[4-(4-{[3-(1-Piperidinyl)propyl]oxy}phenyl)-1-piperidinyl]carbonyl}morpholine (E8)

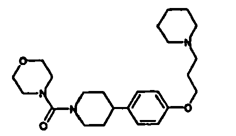

Morpholine-carbonyl chloride (71µl; 0.48mmol) was added to a mixture 1-(3-{[4-(4-piperidinyl)phenyl]oxy}propyl)piperidine (D3) (120mg; 0.4mmol) and diethylaminomethyl-polystyrene (300mg of 3.2 mmol/g) in DCM (5ml). After stirring for 60 minutes the mixture was filtered and the filtrate purified by silica gel chromatography eluting with 1:9:90 mixture of 0.880 ammonia solution:methanol:dichloromethane to afford the title compound (118mg; 62%) MS (ES+), m/e 416 [M+H]⁺.

### Examples 9-10

Examples 9-10 (E9-10) were prepared using the method described for Example 8 substituting morpholine-carbonyl chloride for the appropriate carbonyl chloride indicated in the table.

| **Example** | **Carbonyl chloride** | **Mass Spectrum** |
|---|---|---|
| 1-(1-Piperidinylcarbonyl)4-(4-{[3-(1-piperidinyl)propyl]oxy}phenyl)piperidine (E9) | Piperidine-1-carbonyl chloride | MS (ES+), m/e 414 [M+H]⁺. |
| 4-(4-{[3-(1-Piperidinyl)propyl]oxy}phenyl)-1-(1-pyrrolidinylcarbonyl)piperidine (E10) | Pyrrolidine-1-carbonyl chloride | MS (ES+), m/e400 [M+H]⁺. |

### Example 11

### 1-(4-Fluoro-phenyl)-1-{4-[4-(1-isopropyl-piperidin-4-yloxy)-phenyl]-piperidin-1-yl}-methanone (E11)

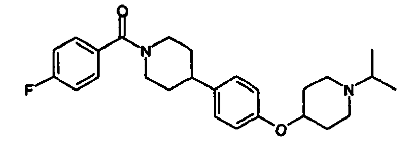

A mixture 4-fluorobenzoic acid (112mg, 0.8mmol), 1-hydroxybenzotriazole hydrate (108mg, 0.8mmol) and N-cyclohexylcarbodiimide-N'-methyl polystyrene (330mg, 0.8mmol, resin loading 1.8mmol/g) in dichloromethane (5ml) was stirred at room temperature for 15 minutes. A solution of 1-(1-methylethyl)-4-{[4-(4-piperidinyl)phenyl] oxy}piperidine (120mg, 0.4mmol) (120mg, 0.4mmol) (D8) in dichloromethane (3ml) was added and the mixture stirred at room temperature for 24 hours. The mixture was filtered through a SCX column eluting with methanol followed by 10% 0.880 ammonia solution in methanol to elute the product. The residue was purified by silica gel chromatography eluting with a mixture of 0.880 ammonia solution:methanol:dichloromethane (1:9:90) to afford the title compound (78mg, 74%); MS(ES+) m/e 425 [M+H]⁺.

### Example 12

### 4-([4-(4-{[1-(1-Methylethyl)-4-piperidinyl]oxy}phenyl)-1-piperidinyl] carbonyl) benzonitrile (E12)

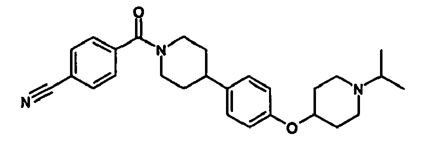

A mixture 4-cyanobenzoic acid (88mg, 0.6mmol), 1-hydroxybenzotriazole hydrate (81 mg, 0.6mmol) and N-cyclohexylcarbodiimide-N'-methyl polystyrene (250mg, 0.6mmol, resin loading 1.8mmol/g) in dichloromethane (4ml) was stirred at room temperature for 60 minutes. A solution of 1-(1-methylethyl)-4-{[4-(4-piperidinyl)phenyl]oxy}piperidine (120mg, 0.4mmol) (D8) in dichloromethane (2ml) was added and the mixture stirred at room temperature for 24 hours. The mixture was filtered through a SCX column eluting with methanol followed by 10% 0.880 ammonia solution in methanol to elute the product. The residue was purified by silica gel chromatography eluting with a mixture of 0.880 ammonia solution:methanol:dichloromethane (1:9:90) to afford the title compound (132mg, 78%); MS(ES+) m/e 432 [M+H]⁺. NMR(CDCl3) δ 1.06 (6H, d), 1.84 (4H, m), 2.00 (4H, m), 2.39 (2H, m), 2.79 (4H, m), 2.87 (H, m), 3.15 (H, m), 3.69 (H, m), 4.27 (H, m), 4.85 (H, m), 6.86 (2H, m), 7.10 (2H, m), 7.54 (2H, m), 7.72 (2H, m)

### Examples 13-18

Examples 13-18 (E13-18) were prepared from 1-(1-methylethyl)-4-{[4-(4-piperidinyl)phenyl]oxy}piperidine (D8) using an analogous method to that described for Example 11, exchanging 4-fluorobenzoic acid for the appropriate acid indicated in the table below:

| **Example** | **Acid** | **Mass Spectrum** |
|---|---|---|
| 1-(1-Methylethyl)-4-{[4-(1-{[4-(1-pyrrolidinylcarbonyl)phenyl]carbonyl}-4-piperidinyl)phenyl]oxy}piperidine (E13) | 4-(1-pyrrolidin-1-yl-methanoyl)-benzoic acid (J. Med. Chem. 46(10), 1845, 2003) | MS (ES+), m/e 504 [M+H]⁺. |
| 1-(1-Methylethyl)-4-({4-[1-(tetrahydro-2H pyran-4-ylcarbonyl)-4-piperidinyl] phenyl}oxy)piperidine (E14) | Tetrahydro-pyran-4-carboxylic acid | MS (ES+), m/e 415 [M+H]⁺. |
| 1-(1-Methylethyl)-4-{[4-(1-{[4-(methylsulfonyl)phenyl]carbonyl}-4-piperidinyl)phenyl]oxy}piperidine (E15) | 4-methanesulfonyl-benzoic acid | MS (ES+), m/e 485 [M+H]⁺. |
| 1-(1-Methylethyl)-4-[{4-{1-[3-(methyloxy)propanoyl]-4-piperidinyl} phenyl)oxy]piperidine (E16) | 3-methoxy-propionic acid | MS (ES+), m/e 389 [M+H]⁺. |
| 4-{[4-(4-{[1-(1-Methylethyl)-4-piperidinyl]oxy}phenyl)1-piperidinyl] carbonyl}pyridine (E17) | isonicotinic acid | MS (ES+), m/e 408 [M+H]⁺. |
| 3-{[4-(4-{[1-(1-Methylethyl)-4-piperidinyl]oxy}phenyl)-1-piperidinyf] carbonyl}pyridine (E18) | nicotinic acid | MS (ES+), m/e 408 [M+H]⁺. |

### Example 19

### 4-{[4-(4-{[1-(1-Methylethyl)-4-piperidinyl]oxy}phenyl)-1-piperidinyl]carbonyl} morpholine (E19)

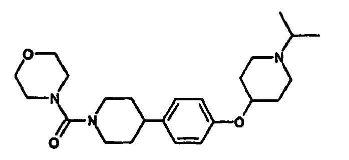

Morpholine-carbonyl chloride (71µl; 0.48mmol) was added to a mixture of 1-(1-methylethyl)-4-{[4-(4-piperidinyl)phenyl]oxy}piperidine (120mg; 0.4mmol) (D8) and diethylaminomethyl-polystyrene (300mg of 3.2 mmol/g) in DCM (5ml). After stirring for 60 minutes the mixture was filtered and the filtrate purified by silica gel chromatography eluting with 1:9:90 mixture of 0.880 ammonia solution:methanol:dichloromethane to afford the title compound (127mg; 78%) MS (ES+), m/e 416 [M+H]⁺.

### Example 20

### 1-(1-Azetidinylcarbonyl)-4-(4-{[1-(1-methylethyl)-4-piperidinyl]oxy}phenyl) piperidine (E20)

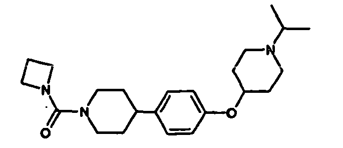

A solution of 1-(1-methylethyl)4-{[4-(4-piperidinyl)phenyl]oxy}piperidine (150mg; 0.5mmol) (D8) in DCM (3ml) was added drop-wise to a stirring solution of phosgene in toluene (2ml of 2M soln; 4mmol). After 30 minutes the solvent was removed *in vacuo* and the residue redissolved in DCM (5ml). This solution was treated with triethylamine (146µl; 1.1mmol) and azetidine (37µl; 0.55mmol) and stirred for 60 minutes at room temperature. The mixture was concentrated *in vacuo* and the residue purified by silica gel chromatography eluting with a mixture of 0.880 ammonia solution:methanol:dichloromethane (1:9:90) to afford the title compound (109mg; 58%) MS (ES+), m/e 386 [M+H]⁺.

### Examples 21-22

Examples 21-22 were prepared from 1-(1-methylethyl)-4-{[4-(4-piperidinyl)phenyl]oxy}piperidine (D8) using an analogous method to that described for Example 20, exchanging azetidine for the appropriate amine indicated in the table below:

| **Example** | **Amine** | **Mass Spectrum** |
|---|---|---|
| 1-(1-Methylethyl)-4-({4-[1-(1-pyrrolidinylcarbonyl)-4-piperidinyl] phenyl}oxy)piperidine (E21) | Pyrrolidine | MS (ES+), m/e 400 [M+H]⁺. |
| 1-(1-Methylethyl)-4-({4-[1-(1-piperidinylcarbonyl)-4-piperidinyl]phenyl}oxy)piperidine (E22) | Piperidine | MS (ES+), m/e 414 [M+H]⁺. |

### Example 23

### 4-{[4-(4-{[1-(1-Methylethyl)-4-piperidinyl]oxy}phenyl)-1-piperidinyl] carbonyl} thiomorpholine 1,1 -dioxide (E23)

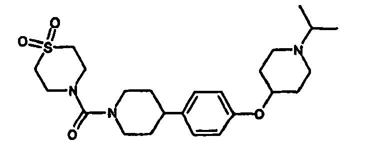

A solution of 1-(1-methylethyl)-4-{[4-(4-piperidinyl)phenyl]oxy}piperidine (150mg; 0.5mmol) (D8) in DCM (3ml) was added drop-wise to a stirring solution of phosgene in toluene (2ml of 2M soln; 4mmol). After 30 minutes the solvent was removed *in vacuo* and the residue redissolved in DCM (5ml). This solution was treated with triethylamine (146µl; 1.1mmol) and thiomorpholine 1,1-dioxide (J. Med. Chem. 37(7), 913 - 923, 1994) (148mg; 0.55mmol) and stirred for 60 minutes at room temperature. Methylisocyanate polystyrene (1.1g of 1.8 mmol/g resin; 2mmol) was added and the mixture stirred at room temperature for 30 minutes. The mixture was filtered and the filtrate was purified by silica gel chromatography eluting with a mixture of 0.880 ammonia solution:methanol:dichloromethane (1:9:90) to afford the title compound (122mg, 53%); MS(ES+) m/e 464 [M+H]⁺.

### Examples 24-29

Examples 24-29 (E24-E29) were prepared from 1-cyclobutyl-4-{[4-(4-piperidinyl)phenyl] oxy}piperidine (D10) using an analogous method to that described for Example 11 (E11), using the appropriate acid indicated in the table below:

| **Example** | **Acid** | **Mass Spectrum** |
|---|---|---|
| 4-[(4-{4-[(1-Cyclobutyl-4-piperidinyl)oxy] phenyl}-1-piperidinyl)carbonyl] benzonitrile (E24) | 4-cyano-benzoic acid | MS (ES+), m/e 444 [M+H]⁺. |
| 1-Cyclobutyl-4-[(4-{1-[(4-fluorophenyl) carbonyl]-4-piperidinyl}phenyl) oxy] piperidine (E25) | 4-fluorobenzoic acid | MS(ES+) m/e 437 [M+H]⁺. |
| 1-Cyclobutyl-4-{[4-(1-{[4-(1-pyrrolidinylcarbonyl)phenyl]carbonyl}-4-piperidinyl)phenyl]oxy}piperidine (E26) | 4-(1-pyrrolidin-1-yl-methanoyl)-benzoic acid (J. Med. Chem. 46(10), 1845, 2003) | MS (ES+), m/e 516 [M+H]⁺. |
| 1 -Cyclobutyl-4-[(4-(l-[3-(methyloxy) propanoyl]-4-piperidinyl) phenyl)oxy] piperidine (E27) | 3-methoxy-propionic acid | MS (ES+), m/e 401 [M+H]⁺. |
| 4-[(4-{4-[(1-Cyclobutyl-4-piperidinyl)oxy] phenyl}-1-piperidinyl)carbonyl]pyridine (E28) | isonicotinic acid | MS (ES+), m/e 420 [M+H]⁺. |
| 3-[(4-{4-[(1-Cyclobutyl-4-piperidinyl)oxy]phenyl}-1-piperidinyl)carbonyl]pyridine (E29) | nicotinic acid | MS (ES+), m/e 420 [M+H]⁺. |

### Example 30

### 4-[(4-{4-[(1-Cyclobutyl-4-piperidinyl)oxy]phenyl}-1-piperidinyl)carbonyl]morpholine (E30)

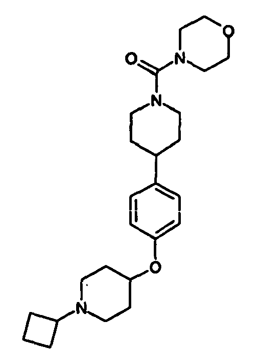

Morpholine-carbonyl chloride (60µl; 0.52mmol) was added to a mixture of 1-cydobutyl-4-([4-(4-piperidinyl)phenyl]oxy)piperidine (148mg; 0.47mmol) (D10) and triethylamine (80µl; 0.56mmol) in DCM (5ml). After stirring for 18 hours mixture was filtered through a SCX column eluting with methanol followed by 10% 0.880 ammonia solution in methanol to elute the product. The product was purified further by silica gel chromatography eluting with a 1:9:90 mixture of 0.880 ammonia solution:methanol:dichloromethane to afford the title compound (175mg, 92%); MS(ES+) m/e 428 [M+H]⁺.

### Example 31

### 1-[(4-Fluorophenyl)carbonyl]-4-(4-{[3-(1-piperidinyl)propyl]oxy}phenyl)-1,2,3,6-tetrahydropyridine (E31)

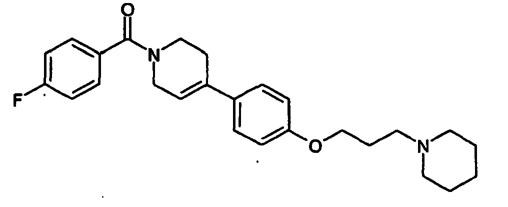

A mixture 4-fluorobenzoic acid (140mg, 1.0mmol), 1-hydroxybenzotriazole hydrate (135mg, 1.0mmol) and N-cyclohexylcarbodiimide-N'-methyl polystyrene (550mg, 1.0mmol, resin loading 1.8mmol/g) in dichloromethane (5ml) was stirred at room temperature for 15 minutes. A solution of 4-(4-{[3-(1-piperidinyl)propyl]oxy}phenyl)-1,2,3,6-tetrahydropyridine (150mg, 0.5mmol) (D12) in dichloromethane (3ml) was added and the mixture stirred at room temperature for 24 hours. The mixture was filtered through a SCX column, eluting with methanol followed by 10% 0.880 ammonia solution in methanol to elute the product. The residue was purified by silica gel chromatography eluting with a 1:9:90 mixture of 0.880 ammonia solution:methanol:dichloromethane to afford the title compound (137mg, 64%); MS(ES+) m/e 423 [M+H]⁺.

### Examples 32-35

Examples 32-35 (E32-E35) were prepared from 4-(4-{[3-(1-piperidinyl)propyl]oxy}phenyl) -1,2,3,6-tetrahydropyridine (D12) using an analogous method to that described for Example 31 using the appropriate acid indicated in the table below

| Example | Acid | Mass Spectrum |
|---|---|---|
| 4-{[4-(4-{[3-(1-Piperidinyl)propyl]oxy} phenyl)-3,6-dihydro-1 (2*H*)-pyridinyl] carbonyl} benzonitrile (E32) | 4-cyano-benzoic acid | MS (ES+), m/e 430 [M+H]⁺. |
| 4-(4-{[3-(1-Piperidinyl)propyl] oxy}phenyl)-1-{[4-{1pyrrolidinylcarbonyl)phenyl]carbonyl}-1,2,3,6-tetrahydropyridine (E33) | 4-(1-pyrrolidin-1-yl-methanoyl)-benzoic acid (J. Med. Chem. 46(10), 1845, 2003) | MS (ES+), m/e 502 [M+H]⁺. |
| 4-(4-{[3-(1-Piperidinyl)propyl] oxy} phenyl)-1-(tetrahydro-2H-pyran-4-ylcarbonyl)-1,2,3,6-tetrahydropyridine (E34) | tetrahydropyran-4-carboxylic acid | MS (ES+), m/e 413 [M+H]⁺. |
| 1-{[4-(Methylsulfonyl)phenyl]carbonyl}-4-(4-{[3-(1-piperidinyl)propyl]oxy} phenyl) -1,2,3,6-tetrahydropyridine (E35) | 4-methanesulfonyl benzoic acid | MS (ES+), m/e 483 [M+H]⁺. |

### Example 36

### 4-{[4-(4-{[3-(1-Piperidinyl)propyl]oxy}phenyl)-3,6-dihydro-1(2H)-pyridinyl]carbonyl} morpholine (E36)

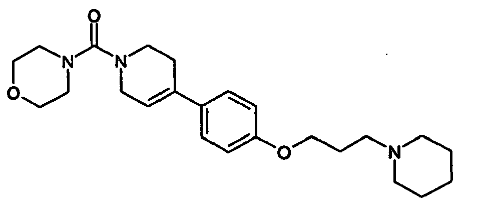

Morpholine-carbonyl chloride (116µl; 0.55mmol) was added to a mixture of 4-(4-{[3-(1-piperidinyl)propyl]oxy}phenyl)-1,2,3,6-tetrahydropyridine (150mg; 0.5mmol) (D12) and diethylaminomethyl-polystyrene (330mg of 3.2 mmol/g) in DCM (5ml). After stirring for 60 minutes the mixture was filtered and the filtrate purified by silica gel chromatography eluting with 1:9:90 mixture of 0.880 ammonia solution:methanol:dichloromethane to afford the title compound (126mg; 62%) MS (ES+), m/e 414 [M+H]⁺.

### Examples 37-38

Examples 37-38 (E37-E38) were prepared from 4-(4-{[3-(1-piperidinyl)propyl]oxy} phenyl)-1 ,2,3,6-tetrahydropyridine (D12) using an analogous method to that described for Example 36, using the appropriate carbonyl chloride indicated in the table below:

| Example | Carbonyl Chloride | Mass Spectrum |
|---|---|---|
| 1-(1-Piperidinylcarbonyl)-4-(4-{[3-(1-piperidinyl)propyl]oxy}phenyl-1,2,3,6-tetrahydropyridine (E37) | Piperidine carbonyl chloride | (ES+), m/e 412 [M+H]⁺. |
| 4-(4-{[3-(1-Piperidinyl)propyf]oxy} phenyl)-1-(1-pyrrolidinylcarbonyl)-1,2,3,6-tetrahydropyridine (E38) | Pyrrolidine carbonyl chloride | (ES+), m/e 398 [M+H]⁺. |

### Examples 39-44

Examples 39-44 (E39-E44) were prepared from 4-(4-{[1-{1-methylethyl)-4-piperidinyl]oxy}phenyl)-1,2,3,6-tetrahydropyridine (D16) using an analogous method to that described for Example 11 (E11), using the appropriate acid indicated in the table below:

| **Example** | **Acid** | **Mass Spectrum** |
|---|---|---|
| 1-[(4-Fluorophenyl)carbonyl]-4-(4-{[1-(1-methylethyl)-4-piperidinyl]oxy}phenyl)-1,2,3,6-tetrahydropyridine (E39) | 4-fluorobenzoic acid | MS (ES+), m/e 423 [M+H]⁺. |
| 4-{[4-(4-{[1-(1-Methylethyl)-4-piperidinyl]oxy}phenyl)-3,6-dihydro-1 (2*H*)-pyridinyl]carbonyl}benzonitrile (E40) | 4-cyano-benzoic acid | MS(ES+) m/e 430 [M+H]⁺. |
| 4-(4-{[1-(1-Methylethyl)-4-piperidinyl]oxy}phenyl)-1-{[4-(1-pyrrolidinylcarbonyl)phenyl]carbonyl}-1,2,3,6-tetrahydropyridine (E41) | 4-(1-pyrrolidin-1-yl-methanoyl)-benzoic acid (J. Med. Chem. 46(10), 1845, 2003) | MS (ES+), m/e 502 [M+H]⁺. |
| 4-(4-{[1-(1-Methylethyl)-4-piperidinyl]oxy} phenyl)-1-(tetrahydro-2H-pyran-4-ylcarbonyl)-1,2,3,6-tetrahydropyridine (E42) | tetrahydropyran-4-carboxylic acid | MS (ES+), m/e 413 [M+H]⁺. |
| 4-(4-{[1-(1-Methylethyl)-4-piperidinyl]oxy}phenyl)-1-{[4-(methylsulfonyl)phenyl]carbonyl}-1,2,3,6-tetrahydropyridine (E43) | 4-methanesulfonyl benzoic acid | MS (ES+), m/e 483 [M+H]⁺. |
| 4-{[4-(4-{[1-(1-Methylethyl)-4-piperidinyl]oxy}phenyl)-3,6-dihydro-1(*2H*)-pyridinyl]carbonyl}pyridine (E44) | isonicotinic acid | MS (ES+), m/e 406 [M+H]⁺. |

### Examples 45-47

Examples 45-47 (E45-E47) were prepared 4-(4-{[1-(1-methytethyl)-4-piperidinyl]oxy}phenyl)-1,2,3,6-tetrahydropyridine (D16) using an analogous method to that described for Example 36, using the appropriate carbonyl chloride indicated in the table below:

| **Example** | **Carbonyl Chloride** | **Mass Spectrum** |
|---|---|---|
| 4-{[4-(4-{[1-(1-Methylethyl)-4-piperidinyl]oxy}phenyl)-3,6-dihydro-1 (2*H*)-pyridinyl]carbonyl}morpholine (E45) | Morpholine carbonyl chloride | MS.(ES+), m/e 414 [M+H]⁺. |
| 4-(4-{[1-(1-Methylethyl)-4-piperidinyl]oxy}phenyl)-1-(1-piperidinylcarbonyl)-1,2,3,6-tetrahydropyridine (E46) | Piperidine carbonyl chloride | MS (ES+), m/e 412 [M+H]⁺. |
| 4-(4-{[1-(1-Methylethyl)-4-piperidinyl] oxy}phenyl)-1-(1-pyrrolidinyl carbonyl)-1,2,3,6-tetrahydropyridine (E47) | Pyrrolidine carbonyl chloride | MS (ES+), m/e 398 [M+H]⁺ . |

### Example 48

### 4-({4-[4-({3-[(2R)-2-Methyl-1-pyrrolidinyl]propyl}oxy)phenyl]-1-piperidinyl} carbonyl)benzonitrile (E48)

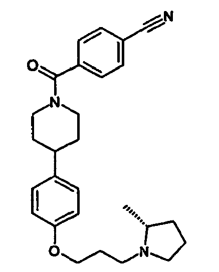

A mixture of 4-[4-({3-[(2*R*)-2-methyl-1-pyrrolidinyl]propyl}oxy)phenyl]piperidine (1.05g; 2 mmol) (D21), triethylamine (1.4 ml; 10mmol) and 4-cyanobenzoyl chloride (0.36g; 2.2 mmol) in dichloromethane (20 ml) was stirred at room temperature for 3 days. The mixture was washed with saturated sodium hydrogen carbonate solution, dried with sodium sulfate and evaporated. The residue was purified by column chromatography on silica eluting with 20-1 dichloromethane - 2M ammonia in methanol to give a yellow oil (0.4g) MS (ES+), m/e 432 [M+H]⁺.

### Example 49

### 4-[4-({3-[(2R)-2-Methyl-1-pyrrolidinyl]propyl}oxy)phenyl]-1-(tetrahydro-2H-pyran-4-ylcarbonyl)piperidine (E49)

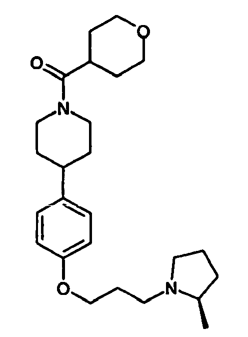

A mixture of 3-({4-[1-(tetrahydro-2*H*-pyran-4-ylcarbonyl)-4-piperidinyl]phenyl}oxy)propyl methanesulfonate (0.15g; 0.35 mmol) (D28), (2R)-2-methylpyrrolidine hydrobromide (0.11g; 0.10 mmol) and potassium carbonate (0.2g; 1.41 mmol) in acetonitrile (20 ml) was heated at 50°C for 18 hours. The mixture was poured into water and extracted with ethyl acetate. The organic extracts were dried with sodium sulfate and evaporated. The residue was purified by column chromatography on silica eluting with 200-10-1 dichloromethane - ethanol -0.88 ammonia solution to give a colourless solid (0.11 g; 76%), MS (ES+), m/e 415 [M+H]⁺.

### Example 50

### 4-[4-({3-[(2R,5R)-2,5-Dimethyl-1-pyrrolidinyl]propyl}oxy)phenyl]-1-(tetrahydro-2H pyran-4-ylcarbonyl)piperidine (E50)

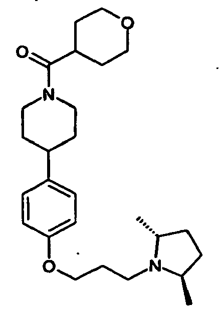

A mixture of 3-({4-[1-(tetrahydro-2*H*-pyran-4-ylcarbonyl)-4-piperidinyl]phenyl} oxy)propyl methanesulfonate (0.15g; 0.35 mmol) (D28), (2R,5R)-2,5-dimethylpyrrolidine hydrobromide (0.11g; 0.64 mmol) and potassium carbonate (0.2g; 1.41 mmol) in acetonitrile (20 ml) was heated at 50°C for 18 hours. The mixture was poured into water and extracted with ethyl acetate. The organic extracts were dried with sodium sulfate and evaporated. The residue was purified by column chromatography on silica eluting with 300-10-1 dichloromethane - ethanol - 0.88 ammonia solution to give a colourless solid (60 mg; 40%), MS (ES+), m/e 429 [M+H]⁺.

### Example 51

### 5-([4-(4-{[1-(1-Methylethyl)-4-piperidinyl]oxy}phenyl)-1-piperidinyl]carbonyl}-2-pyridinecarbonitrile (E51)

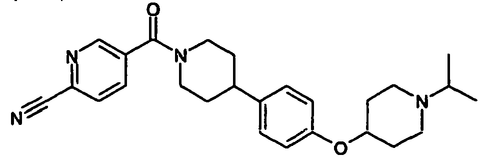

A mixture of 6-cyano-3-pyridine carboxylic acid (71 mg, 0.48 mmol), 1-hydroxybenzotriazole hydrate (75 mg, 0.48 mmol) and N-cyctohexylcarbodiimide-N'-methyl polystyrene (133 mg, 0.48 mmol, resin loading 1.8mmol/g) in dichloromethane (5ml) was stirred at room temperature for 15 minutes. A solution of 1-(1-methylethyl)-4-{[4-(4-piperidinyl)phenyl] oxy}piperidine (120mg, 0.4mmol) (75 mg, 0.24mmol) (D8) in dichloromethane (3ml) was added and the mixture stirred at room temperature for 24 hours. The mixture was filtered through a SCX column eluting with methanol followed by 10% 0.880 ammonia solution in methanol to elute the product. The residue was purified by silica gel chromatography eluting with a mixture of 0.880 ammonia solution:methanol:dichloromethane (1:9:90) to afford the title compound (28 mg. 27%); MS(ES+) m/e 433 [M+H]⁺. NMR(CDCl₃) δ 1.06 (6H, d), 1.83 (4H, m), 2.00 (4H, m), 2.39 (2H, m), 2.79 (4H, m), 2.91 (H, m), 3.26 (H, m), 3.71 (H, m), 4.27 (H, m), 4.86 (H, m), 6.86 (2H, m), 7.09 (2H, m), 7.77 (H, d), 7.92 (H, d), 8.78 (H, s)

### Example 52

### 5-{[4-(4-{[1-(1-Methylethyl)-4-piperidinyl]oxy}phenyl)-1-piperidinyl]carbonyl}-2-(trifluoromethyl)pyridine (E52)

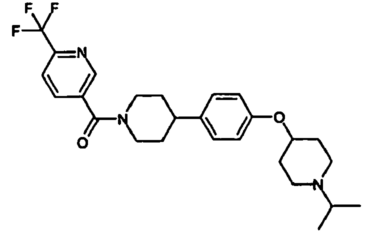

A mixture of 6-(trifluoromethyl)-3-pyridinecarboxylic acid (76 mg, 0.40 mmol), 1-hydroxybenzotriazole hydrate (107 mg, 0.79 mmol) and N-cyclohexylcarbodiimide-N'-methyl polystyrene (464 mg, 0.79 mmol, resin loading 1.7 mmol/g) in dichloromethane (5ml) was stirred at room temperature for 15 minutes. A solution of 1-(1-methylethyl)-4-{[4-(4-piperidinyl)phenyl] oxy}piperidine (120mg, 0.4mmol) (100 mg, 0.33 mmol) (D8) in dichloromethane (3ml) was added and the mixture stirred at room temperature for 24 hours. The mixture was filtered through a SCX column eluting with methanol followed by 10% 0.880 ammonia solution in methanol to elute the product. The residue was purified by silica gel chromatography eluting with a mixture of 0.880 ammonia solution :methanol: dichloromethane (1:9:90) to afford the title compound (55 mg, 35%); MS(ES+) m/e 476 [M+H]⁺. NMR(CDCl₃) δ 1.07 (6H, d), 1.83 (4H, m), 2.02 (4H, m), 2.39 (2H, m), 2.77 (4H, m), 2.91 (H, m), 3.23 (H, m), 3.74 (H, m), 4.27 (H, m), 4.88 (H, m), 6.86 (2H, m), 7.10 (2H, m), 7.76 (H, d), 7.96 (H, d), 8.80 (H, s)

### Examples 53-56

Examples 53-56 (E53-56) were prepared from 1-(1-methylethyl)-4-{[4-{4-pipendinyl)phenyl]oxy}pipendine (D8) using an analogous method to that described for Example 11, exchanging 4-fluorobenzoic acid for the appropriate acid indicated in the table below:

| **Example** | **Acid** | **Mass Spectrum** |
|---|---|---|
| 2-{[4-(4-{[1-(1-Methylethyl)-4-piperidinyl] oxy}phenyl)-1-piperidinyl]carbonyl} pyrazine (E53) | 2-Pyrazinecarboxylic acid | MS (ES+), m/e 409 [M+H]⁺. |
| 3-{[4-(4-{[1-(1-Methylethyl)-4-piperidinyl] oxy}phenyl)-1-piperidinyl]carbonyl} benzonitrile (E54) | 3-Cyano benzoic acid | MS (ES+), m/e 432 [M+H]⁺. |
| 1-(1-Methylethyl)-4-{[4-(1-{[4-(trifluoromethyl)phenyl]carbonyl}-4-piperidinyl)phenyl]oxy}piperidine (E55) | 4-Trifluoromethyl benzoic acid | MS (ES+), m/e 475 [M+H]⁺. |
| 6-{[4-(4-{[1-(1-Methylethyl)-4-piperidinyl] oxy}phenyl)-1-piperidinyl]carbonyl} quinoxaline (E56) | 6-quinoxaline carboxylic acid | MS (ES+), m/e 459 [M+H]⁺. |

All publications, including but not limited to patents and patent applications, cited in this specification are herein incorporated by reference as if each individual publication were specifically and individually indicated to be incorporated by reference herein as though fully set forth.

### Biological Data

A membrane preparation containing histamine H3 receptors may be prepared in accordance with the following procedures:

### (i) Generation of histamine H3 cell line

DNA encoding the human histamine H3 gene was cloned into a holding vector, pCDNA3.1 TOPO (InVitrogen) and its cDNA was isolated from this vector by restriction digestion of plasmid DNA with the enzymes BamH1 and Not-1 and ligated into the inducible expression vector pGene (InVitrogen) digested with the same enzymes. The GeneSwitch^{™} system (a system where in transgene expression is switched off in the absence of an inducer and switched on in the presence of an inducer) was performed as described in US Patent nos: 5,364,791; 5,874,534; and 5,935,934. Ligated DNA was transformed into competent DH5α *E. coli* host bacterial cells and plated onto Luria Broth (LB) agar containing Zeocin^{™} (an antibiotic which allows the selection of cells expressing the *sh ble* gene which is present on pGene and pSwitch) at 50µg ml⁻¹. Colonies containing the re-ligated plasmid were identified by restriction analysis. DNA for transfection into mammalian cells was prepared from 250ml cultures of the host bacterium containing the pGeneH3 plasmid and isolated using a DNA preparation kit (Qiagen Midi-Prep) as per manufacturers guidelines (Qiagen). CHO K1 cells previously transfected with the pSwitch regulatory plasmid (InVitrogen) were seeded at 2x10e6 cells per T75 flask in Complete Medium, containing Hams F12 (GIBCOBRL, Life Technologies) medium supplemented with 10% v/v dialysed foetal bovine serum, L-glutamine, and hygromycin (100µg ml⁻¹), 24 hours prior to use. Plasmid DNA was transfected into the cells using Lipofectamine plus according to the manufacturers guidelines (InVitrogen). 48 hours post transfection cells were placed into complete medium supplemented with 500µg ml⁻¹ Zeocin^{™}. 10-14 days post selection 10nM Mifepristone (InVitrogen), was added to the culture medium to induce the expression of the receptor. 18 hours post induction cells were detached from the flask using ethylenediamine tetra-acetic acid (EDTA; 1:5000; InVitrogen), following several washes with phosphate buffered saline pH 7.4 and resuspended in Sorting Medium containing Minimum Essential Medium (MEM), without phenol red, and supplemented with Earles salts and 3% Foetal Clone II (Hyclone). Approximately 1 x 10e7 cells were examined for receptor expression by staining with a rabbit polyclonal antibody, 4a, raised against the N-terminal domain of the histamine H3 receptor, incubated on ice for 60 minutes, followed by two washes in sorting medium. Receptor bound antibody was detected by incubation of the cells for 60 minutes on ice with a goat anti rabbit antibody, conjugated with Alexa 488 fluorescence marker (Molecular Probes). Following two further washes with Sorting Medium, cells were filtered through a 50µm Filcon^{™} (BD Biosciences) and then analysed on a FACS Vantage SE Flow Cytometer fitted with an Automatic Cell Deposition Unit. Control cells were non-induced cells treated in a similar manner. Positively stained cells were sorted as single cells into 96-well plates, containing Complete Medium containing 500µg ml⁻¹ Zeocin^{™} and allowed to expand before reanalysis for receptor expression via antibody and ligand binding studies. One clone, 3H3, was selected for membrane preparation.

### (ii) Membrane preparation from cultured cells

All steps of the protocol are carried out at 4°C and with pre-cooled reagents. The cell pellet is resuspended in 10 volumes of buffer A2 containing 50mM N-2-hydroxyethylpiperazine-N'-2-ethanesulfonic acid (HEPES) (pH 7.40) supplemented with 10e-4M leupeptin (acetyl-leucyl-leucyl-arginal; Sigma L2884), 25µg/ml bacitracin (Sigma B0125), 1mM ethylenediamine tetra-acetic acid (EDTA), 1mM phenylmethylsulfonyl fluoride (PMSF) and 2x10e-6M pepstain A (Sigma). The cells are then homogenised by 2 x 15 second bursts in a 1 litre glass Waring blender, followed by centrifugation at 500g for 20 minutes. The supernatant is then spun at 48,000g for 30 minutes. The pellet is resuspended in 4 volumes of buffer A2 by vortexing for 5 seconds, followed by homogenisation in a Dounce homogeniser (10-15 strokes). At this point the preparation is aliquoted into polypropylene tubes and stored at -70°C.

Compounds of the invention may be tested for *in vitro* biological activity in accordance with the following assays:

### (I) Histamine H3 binding assay

For each compound being assayed, in a white walled clear bottom 96 well plate, is added:-
(a) 10µl of test compound (or 10µl of iodophenpropit (a known histamine H3 antagonist) at a final concentration of 10mM) diluted to the required concentration in 10% DMSO;
(b) 10µl ¹²⁵I 4-[3-(4-iodophenylmethoxy)propyl]-1H-imidazolium (iodoproxyfan) (Amersham; 1.85MBq/µl or 50µCi/ml; Specific Activity ~2000Ci/mmol) diluted to 200pM in assay buffer (50mM Tris(hydroxymethyl)aminomethane buffer (TRIS) pH 7.4, 0.5mM ethylenediamine tetra-acetic acid (EDTA)) to give 20pM final concentration; and
(c) 80µl bead/membrane mix prepared by suspending Scintillation Proximity Assay (SPA) bead type WGA-PVT at 100mg/ml in assay buffer followed by mixing with membrane (prepared in accordance with the methodology described above) and diluting in assay buffer to give a final volume of 80µl which contains 7.5µg protein and 0.25mg bead per well - mixture was pre-mixed at room temperature for 60 minutes on a roller. The plate is shaken for 5 minutes and then allowed to stand at room temperature for 3-4 hours prior to reading in a Wallac Microbeta counter on a 1 minute normalised tritium count protocol. Data was analysed using a 4-parameter logistic equation.

### (II) Histamine H3 functional antagonist assay

For each compound being assayed, in a white walled clear bottom 96 well plate, is added:-
(a) 10µl of test compound (or 10µl of guanosine 5'- triphosphate (GTP) (Sigma) as non-specific binding control) diluted to required concentration in assay buffer (20mM N-2-Hydroxyethylpiperazine-N'-2-ethanesutfonic acid (HEPES) + 100mM NaCl + 10mM MgCl₂, pH7.4 NaOH);
(b) 60µl bead/membrane/GDP mix prepared by suspending wheat germ agglutinin-polyvinyltoluene (WGA-PVT) scintillation proximity assay (SPA) beads at 100mg/ml in assay buffer followed by mixing with membrane (prepared in accordance with the methodology described above) and diluting in assay buffer to give a final volume of 60µl which contains 10µg protein and 0.5mg bead per well- mixture is pre-mixed at 4°C for 30 minutes on a roller and just prior to addition to the plate, 10µM final concentration of guanosine 5' diphosphate (GDP) (Sigma; diluted in assay buffer) is added; The plate is incubated at room temperature to equilibrate antagonist with receptor/beads by shaking for 30 minutes followed by addition of:
(c) 10µl histamine (Tocris) at a final concentration of 0.3µM; and
(d) 20µl guanosine 5' [y35-S] thiotriphosphate, triethylamine salt (Amersham; radioactivity concentration = 37kBq/µl or 1 mCi/ml; Specific Activity 1160Ci/mmol) diluted to 1.9nM in assay buffer to give 0.38nM final.
   The plate is then incubated on a shaker at room temperature for 30 minutes followed by centrifugation for 5 minutes at 1500 rpm. The plate is read between 3 and 6 hours after completion of centrifuge run in a Wallac Microbeta counter on a 1 minute normalised tritium count protocol. Data is analysed using a 4-parameter logistic equation. Basal activity used as minimum i.e. histamine not added to well.

### Results

The compounds of Examples E1-E53 were tested in the histamine H3 functional antagonist assay and exhibited pK_{b} values > 8.0, more particularly, the compounds of Examples E1-E38 exhibited pK_{b} values > 8.5, most particularly, the compounds of Examples E12, E51 and E52 exhibited pK_{b} values > 9.0.

## Claims

1. A compound of formula (I): 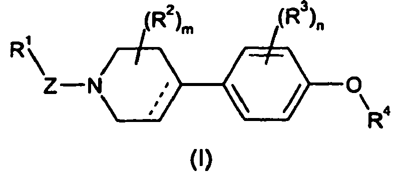 wherein:
R¹ represents -C₁₋₆ alkyl-O-C₁₋₆ alkyl, -C₃₋₈ cycloalkyl, aryl, heterocyclyl, heteroaryl, - C₁₋₆ alkyl-aryl, -C₁₋₆ alkyl-heteroaryl, -C₁₋₆ alkyl-heterocyclyl, -aryl-X-aryl, -aryl-X-heteroaryl, -aryl-X-heterocyclyl,- heteroaryl-X-aryl, -heteroaryl-X-heteroaryl, - heteroaryl-X-heterocyclyl, -heterocyclyl-X-aryl, -heterocyclyl-X-heteroaryl or - heterocyclyl-X-heterocyclyl,
wherein said C₁₋₆ alkyl, C₃₋₈ cycloalkyl, aryl, heteroaryl and heterocyclyl groups of R¹ may be optionally substituted by one or more (eg. 1, 2 or 3) substituents which may be the same or different, and which are selected from the group consisting of halogen, hydroxy, cyano, nitro, oxo, haloC₁₋₆ alkyl, polyhaloC₁₋₆ alkyl, haloC₁₋₆ alkoxy, polyhaloC₁₋₆ alkoxy, C₁₋₆ alkyl, C₁₋₆ alkoxy, C₁₋₆ alkylthio, C₁₋₆ alkoxyC₁₋₆ alkyl, C₃₋₇ cycloalkylC₁₋₆ alkoxy, C₁₋₆ alkanoyl, C₁₋₆ alkoxycarbonyl, C₁₋₆ alkylsulfonyl, C₁₋₆ alkylsulfinyl, C₁₋₆ alkylsulfonyloxy, C₁₋₆ alkylsulfonylC₁₋₆ alkyl, C₁₋₆ alkylsuffonamidoC₁₋₆ alkyl, C₁₋₆ alkylamidoC₁₋₆ alkyl, arylsulfonyl, arylsulfonyloxy, aryloxy, arylsulfonamido, arylcarboxamido, aroyl, or a group NR¹⁵R¹⁶, -CONR¹⁵R¹⁶,-NR¹⁵COR¹⁶, -NR¹⁵SO₂R¹⁶ or -SO₂NR¹⁵R¹⁶, wherein R¹⁵ and R¹⁶ independently represent hydrogen or C₁₋₆ alkyl or together form a heterocyclic ring;
X represents a bond, O, CO, OCH₂, CH₂O or SO₂;
Z represents CO, CONR¹⁰ or SO₂;
R¹⁰ represents hydrogen, C₁₋₆ alkyl, -C₃₋₈ cycloalkyl, aryl, heterocyclyl, heteroaryl;
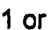 represents a single or a double bond;
m and n independently represent 0, 1 or 2;
R² represents hydrogen, C₁₋₆ alkyl or C₁₋₆ alkoxy;
R³ represents halogen, C₁₋₆ alkyl, hydroxy, C₁₋₆ alkoxy, cyano, amino, -COC₁₋₆ alkyl, - SO₂C₁₋₆ alkyl or trifluoromethyl;
R⁴ represents -(CH₂)_{q}-NR¹¹R¹² or a group of formula (i): 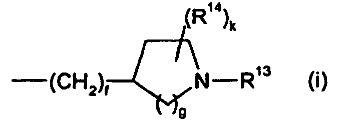 wherein q represents 3 or 4;
-NR¹¹R¹² represents a heterocyclic group, optionally substituted by one or more R¹⁷ groups;
R¹³ represents C₁₋₆ alkyl, C₃₋₈ cycloalkyl, -C₁₋₆ alkyl-C₁₋₆ alkoxy, -C₁₋₆ alkyl-C₃₋₈ cycloalkyl;
R¹⁴ and R¹⁷ independently represent halogen, C₁₋₆ alkyl, haloalkyl, OH or C₁₋₆ alkoxy;
f is 0 or 1;
g is 1 or 2;
k is 0, 1 or 2;
or a pharmaceutically acceptable salt thereof.

2. A compound as defined in claim 1 wherein R¹ represents:
- aryl optionally substituted by 1 or 2 halogen, haloC₁₋₆ alkyl, cyano or SO₂Me groups;
- aryl-X-heterocyclyl;
- heteroaryl optionally substituted by 1 or 2 haloC₁₋₆ alkyl or cyano groups;
- heterocyclyl optionally substituted by 1 or 2 oxo groups; or
- C₁₋₆ alkyl-O-C₁₋₆ alkyl.

3. A compound as defined in claim 2 wherein R¹ represents tetrahydropyranyl, 4-cyanophenyl, 2-cyanopyridin-3-yl or 2-trifluoromethylpyridin-3-yl.

4. A compound as defined in claim 3 wherein R¹ represents 4-cyanophenyl.

5. A compound as defined in any of claims 1 to 4 wherein X and Z both represent CO.

6. A compound as defined in any of claims 1 to 5 wherein 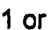 represents a single bond.

7. A compound as defined in any of claims 1 to 6 wherein m and n both represent 0.

8. A compound as defined in any of claims 1 to 7 wherein R⁴ represents -(CH₂)_{q}-NR¹¹R¹², q represents 3 and -NR¹¹R¹² represents N-piperidinyl or N-pyrrolidinyl optionally substituted by 1 or 2 C₁₋₆ alkyl groups or R⁴ represents a group of formula (i) wherein f and k both represent 0, g represents 2 and R¹³ represents C₁₋₆ alkyl or C₃₋₈ cycloalkyl.

9. A compound as defined in claim 8 wherein R⁴ represents a group of formula (i) wherein f and k both represent 0, g represents 2 and R¹³ represents i-propyl.

10. A compound which is:
4-(4-{[3-(1-Piperidinyl)propyl]oxy}phenyl)-1-(tetrahydro-2*H*-pyran-4-ylcarbonyl)piperidine;
4-{[4-(4-{[3-(1-Piperidinyl)propyl]oxy}phenyl)-1-piperidinyl]carbonyl}benzonitrile;
4-{[4-(4-{[3-(1-Piperidinyl)propyl]oxy}phenyl)-1-piperidinyl]carbonyl}pyridine;
**4-(4-{[3-(1-Piperidinyl)propyl]oxy}phenyl)-1-{[4-(1-pyrrolidinylearbonyl)phenyl]** carbonyl} piperidine;
1-{[4-(Methylsulfonyl)phenyl]carbonyl}-4-(4-{[3-(1-piperidinyl) propyl] oxy} phenyl) piperidine;
1-[(4-Fluorophenyl)carbonyl]-4-(4-{[3-(1-piperidinyl)propyl]oxy}phenyl)piperidine;
3-{[4-(4-{[3-(1-Piperidinyl)propyl]oxy}phenyl)-1-piperidinyl]carbonyl}pyridine;
4-{[4-(4-{[3-(1-Piperidinyl)propyl]oxy}phenyl)-1-piperidinyl]carbonyl}morpholine;
1 -(1 1-Piperidinylcarbonyl)-4-(4-{[3-(1-piperidinyl)propyl]oxy}phenyl)piperidine;
4-(4-{[3-(1-Piperidinyl)propyl]oxy}phenyl)-1-(1-pyrrolidinylcarbonyl)piperidine;
1-(4-Fluoro-phenyl)-1-{4-[4-(1-isopropyl-piperidin-4-yloxy)-phenyl]-piperidin-1-yl}-methanone;
1-(1-Methylethyl)-4-{[4-(1-{[4-(1-pyrrolidinylcarbonyl)phenyl]carbonyl}-4-piperidinyl)phenyl]oxy}piperidine;
1-(1-Methylethyl)-4-({4-[1-(tetrahydro-2*H*-pyran-4-ylcarbonyl)-4-piperidinyl] phenyl}oxy)piperidine;
1-(1-Methylethyl)-4-{[4-(1-{[4-(methylsulfonyl)phenyl]carbonyl}-4-piperidinyl)phenyl]oxy}piperidine;
1 -(1 -Methylethyl)-4-[(4-{1-[3-(methyloxy)propanoyl]-4-piperidinyl} phenyl)oxy]piperidine;
4-{[4-(4-{[1-(1-Methylethyl)-4-piperidinyl]oxy}phenyl)-1-piperidinyl] carbonyl}pyridine;
3-{[4-(4-{[1-(1-Methylethyl)-4-piperidinyl]oxy}phenyl)-1-piperidinyl] carbonyl}pyridine;
4-{[4-{4-{[1-(1-Methylethyl)-4-piperidinyl]oxy}phenyl)-1-piperidinyl]carbonyl} morpholine;
1-(1-Azetidinylcarbonyl)-4-(4-{[1-(1-methylethyl)-4-piperidinyl]oxy}phenyl) piperidine;
1-(1-Methylethyl)-4-({4-[1-(1-pyrrolidinylcarbonyl)-4-piperidinyl] phenyl}oxy)piperidine;
1-(1-Methylethyl)-4-({4-[1-(1-piperidinylcarbonyl)-4-piperidinyl]phenyl}oxy)piperidine;
4-{[4-(4-{[1-(1-Methylethyl)-4-piperidinyl]oxy}phenyl)-1-piperidinyl] carbonyl} thiomorpholine 1, 1-dioxide;
4-[(4-{4-[(1-Cyclobutyl-4-piperidinyl)oxy] phenyl}-1-piperidinyl)carbonyl] benzonitrile;
1-Cyclobutyl-4-[(4-{1-[(4-fluorophenyl) carbonyl]-4-piperidinyl}phenyl) oxy] piperidine;
1-Cyclobutyl-4-{[4-(1-{[4-(1-pyrrolidinylcarbonyl)phenyl]carbonyl}-4-piperidinyl)phenyl]oxy}piperidine;
1-Cyclobutyl-4-[(4-{1-[3-(methyloxy) propanoyl]-4-piperidinyl} phenyl)oxy] piperidine;
4-[(4-{4-[(1-Cyclobutyl-4-piperidinyl)oxy] phenyl}-1-piperidinyl)carbonyl]pyridine;
3-[(4-{4-[(1-Cyclobutyl-4-piperidinyl)oxy]phenyl}-1-piperidinyl)carbonyl]pyridine;
4-[(4-{4-[(1-Cyclobutyl-4-piperidinyloxy]phenyl}-1-piperidinyl)carbonyl]morpholine;
1-[(4-Fluorophenyl)carbonyl]-4-(4-{[3-(1-piperidinyl)propyl]oxy}phenyl)-1,2,3,6-tetrahydropyridine;
4-{[4-(4-{[3-(1-Piperidinyl)propyl]oxy}phenyl)-3,6-dihydro-1 (2H)-pyridinyl] carbonyl} benzonitrile;
4-(4-{[3-(1-Piperidinyl)propyl] oxy}phenyl)-1-{[4-(1-pyrrolidinylcarbonyl)phenyl]carbonyl}-1,2,3,6-tetrahydropyridine;
4-(4-{[3-(1-Piperidinyl)propyl] oxy} phenyl)-1-(tetrahydro-2*H*-pyran-4-ylcarbonyl)-1,2,3,6-tetrahydropyridine;
1-{[4-(Methylsulfonyl)phenyl]carbonyl}-4-(4-{[3-(1-Piperidinyl)propyl]oxy} phenyl) - 1,2,3,6-tetrahydropyridine;
4-{[4-(4-{[3-(1-Piperidinyl)propyl]oxy}phenyl)-3,6-dihydro-1(2*H*)-pyridinyl]carbonyl} morpholine;
1-(1-Piperidinylcarbonyl)-4-(4-{[3-(1-piperidinyl)propyl]oxy}phenyl)-1,2,3,6-tetrahydropyridine;
4-(4-{[3-(1-Piperidinyl)propyl]oxy} phenyl)-1-(1-pyrrolidinylcarbonyl)-1,2,3,6-tetrahydropyridine;
1-[(4-Fluorophenyl)carbonyl]-4-(4-{[1-(-methylethyl)-4-pipendinyl]oxy}phenyl)-1,2,3,6-tetrahydropyridine;
4-{[4-(4-{[1-(-Methylethyl)-4-piperidinyl]oxy}phenyl)-3.6-dihydro-1 (*2H)-*pyridinyl]carbonyl}benzonitrile;
4-(4-{[1-(1-Methylethyl)-4-piperidinyl]oxy}phenyl)-1-{[4-(1-pyrrolidinylcarbonyl)phenyl]carbonyl}-1,2,3,6-tetrahydropyridine;
4-(4-{[1-(1-Methylethyl)-4-piperidinyl]oxy} phenyl)-1-(tetrahydro-2*H*-pyran-4-ylcarbonyl)-1,2,3,6-tetrahydropyridine;
4-(4-{[1-(1-Methylethyl)-4-piperidinyl]oxy}phenyl)-1-{[4-(methylsulfonyl)pheny]carbonyl}-1,2,3,6-tetrahydropyridine;
4-{[4-(4-{[1-(1-Methylethyl)-4-piperidinyl]oxy}phenyl)-3,6-dihydro-1 (*2H*)-pyridinyl]carbonyl}pyridine;
4-{[4-(4-{[1-(1-Methylethyl)-4-piperidinyl]oxy}phenyl)-3,6-dihydro-1 (*2H*)-pyridinyl]carbonyl}morpholine;
4-(4-{[1-(1-Methylethyl)-4-piperidinyl]oxy}phenyl)-1-(1-piperidinylcarbonyl)-1,2,3,6-tetrahydropyridine;
4-(4-{[1-(1-Methylethyl)-4-piperidinyl] oxy}phenyl)-1-(1-pyrrolidinyl carbonyl)-1,2,3,6-tetrahydropyridine;
4-({4-[4-({3-[(2R)-2-Methyl-1-pyrrolidinyl]propyl}oxy)phenyl]-1-piperidinyl} carbonyl)benzonitrile;
4-[4-({3-[(2*R*)-2-Methyl-1-pyrrolidinyl]propyl}oxy)phenyl]-1-(tetrahydro-2H-pyran-4-ylcarbonyl)piperidine;
4-[4-({3-[(2*R*,5*R*)-2,5-Dimethyl-1-pyrrolidinyl]propyl}oxy)phenyl]-1-(tetrahydro-*2H-*pyran-4-ylcarbonyl)piperidine;
2-{[4-(4-{[1-(1-Methylethyl)-4-piperidinyl] oxy}phenyl)-1-piperidinyl]carbonyl} pyrazine;
3-{[4-(4-{[1-(1-Methylethyl)-4-piperidinyl] oxy}phenyl)-1-piperidinyl]carbonyl} benzonitrile;
1-(1-Methylethyl)-4-{[4-(1-{[4-(trifluoromethyl)phenyl]carbonyl}-4-piperidinyl)phenyl]oxy}piperidine;
6-{[4-(4-{[1-(1-Methylethyl)-4-piperidinyl] oxy}phenyl)-1-piperidinyl]carbonyl} quinoxaline;
or a pharmaceutically acceptable salt thereof.

11. A compound which is:
5-{[4-(4-{[1-(1-Methylethyl)-4-piperidinyl] oxy}phenyl)-1-piperidinyl]carbonyl}-2-pyridinecarbonitrile; or
5-{[4-(4-{[1-(1-Methylethyl)-4-piperidinyl] oxy}phenyl)-1-piperidinyl]carbonyl}-2-(trifluoromethyl)pyridine;
or a pharmaceutically acceptable salt thereof.

12. A compound which is:
4-{[4-(4-{[1-(1-Methylethyl)-4-piperidinyl]oxy}phenyl)-1-piperidinyl] carbonyl} benzonitrile or a pharmaceutically acceptable salt thereof.

13. A compound which is:
1-(1-Methylethyl)-4-{[4-(1-{[4-{1-pyrrolidinylcarbonyl)phenyl]carbonyl}-4-piperidinyl)phenyl]oxy}piperidine or a pharmaceutically acceptable salt thereof.

14. A pharmaceutical composition which comprises a compound of formula (I) as defined in any of claims 1 to 13 or a pharmaceutically acceptable salt thereof and a pharmaceutically acceptable carrier or excipient.

15. A compound as defined in any of claims 1 to 13 or a pharmaceutically acceptable salt thereof for use in therapy.

16. A compound as defined in any of claims 1 to 13 or a pharmaceutically acceptable salt thereof for use in the treatment of neurological diseases.

17. A compound as defined in any of claims 1 to 13 or a pharmaceutically acceptable salt thereof for use in the treatment of obesity, asthma, allergic rhinitis, nasal congestion, chronic obstructive pulmonary disease and gastro-intestinal disorders.

18. Use of a compound as defined in any of claims 1 to 13 or a pharmaceutically acceptable salt thereof in the manufacture of a medicament for the treatment of neurological diseases.

19. Use of a compound as defined in any of claims 1 to 13 or a pharmaceutically acceptable salt thereof in the manufacture of a medicament for the treatment of obesity, asthma, allergic rhinitis, nasal congestion, chronic obstructive pulmonary disease and gastro-intestinal disorders.

20. A process for the preparation of a compound of formula (I) or a pharmaceutically acceptable salt thereof, which process comprises:
(a) preparing a compound of formula (I) wherein Z represents CO which comprises reacting a compound of formula (II) 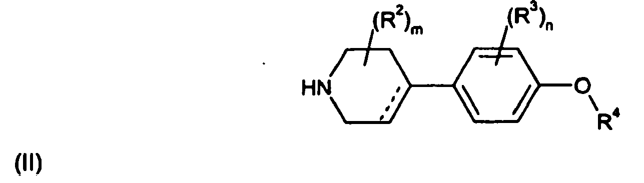 or an optionally activated or protected derivative thereof, wherein 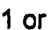 , R², R³, R⁴, m and n are as defined in claim 1, with a compound of formula R¹-CO-L¹, wherein R¹ is as defined in claim 1 and L¹ represents a suitable leaving group such as a suitable halogen atom, or a hydroxyl group; or
(b) preparing a compound of formula (1) wherein Z represents SO₂ which comprises reacting a compound of formula (II), with a compound of formula R¹-SO₂-L², wherein R¹ is as defined in claim 1 and L² represents a suitable leaving group, such as a suitable halogen atom (eg. chlorine); or
(c) preparing a compound of formula (I) wherein Z represents CONH which comprises reacting a compound of formula (II), with a compound of formula R¹-N=C=O, wherein R¹ is as defined in claim 1; or
(d) preparing a compound of formula (I) wherein Z represents CONR¹⁰ which comprises reacting a compound of formula (II), with a compound of formula R¹R¹⁰N-L³, wherein R¹ and R¹⁰ are as defined in claim 1 and L³ represents hydrogen or COCl; or
(e) deprotecting a compound of formula (I) or converting groups which are protected; and optionally thereafter
(f) interconversion to other compounds of formula (1).

## Patentansprüche

1. Verbindung der Formel(I): 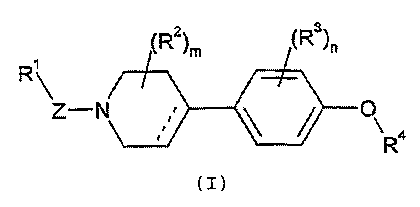 worin:
R¹ -C₁₋₆-Alkyl-O-C₁₋₆-alkyl, -C₃₋₈-Cycloalkyl, Aryl, Heterocyclyl, Heteroaryl, -C₁₋₆-Alkyl-aryl, -C₁₋₆-Alkyl-heteroaryl, -C₁₋₆-Alkyl-heterocyclyl, -Aryl-X-aryl, -Aryl-X-heteroaryl, -Aryl-X-heterocyclyl, -Heteroaryl-X-aryl, -Heteroaryl-X-heteroaryl, -Heteroaryl-X-heterocyclyl, -Heterocyclyl-X-aryl, -Heterocyclyl-X-heteroaryl oder -Heterocyclyl-X-heterocyclyl darstellt,
worin die -C₁₋₆-Alkyl-, -C₃₋₈-Cycloalkyl-, Aryl-, Heteroaryl- und Heterocyclyl-Gruppen von R¹ gegebenenfalls mit einem oder mehreren (z. B. 1, 2 oder 3) Substituenten substituiert sein können, die gleich oder verschieden sein können, und die aus der Gruppe ausgewählt sind, die aus Halogen, Hydroxy, Cyano, Nitro, Oxo, Halogen-C₁₋₆-alkyl, Polyhalogen-C₁₋₆-alkyl, Halogen-C₁₋₆-alkoxy, Polyhalogen-C₁₋₆-alkoxy, C₁₋₆-Alkyl, C₁₋₆-Alkoxy, C₁₋₆-Alkylthio, C₁₋₆-Alkoxy-C₁₋₆-alkyl, C₃₋₇-Cycloalkyl-C₁₋₆-alkoxy, C₁₋₆-Alkanoyl, C₁₋₆-Alkoxycarbonyl, C₁₋₆-Alkylsulfonyl, C₁₋₆-Alkylsulfinyl, C₁₋₆-Alkylsulfonyloxy, C₁₋₆-Alkylsulfonyl-C₁₋₆-alkyl, C₁₋₆-Alkylsulfonamido-C₁₋₆-alkyl, C₁₋₆-Alkylamido-C₁₋₆-alkyl, Arylsulfonyl, Arylsulfonyloxy, Aryloxy, Arylsulfonamido, Arylcarboxamido, Aroyl oder einer Gruppe NR¹⁵R¹⁶, -CONR¹⁵R¹⁶, -NR¹⁵COR¹⁶, -NR¹⁵SO₂R¹⁶ oder -SO₂NR¹⁵R¹⁶ besteht, worin R¹⁵ und R¹⁶ unabhängig voneinander Wasserstoff oder C₁₋₆-Alkyl darstellen oder zusammen einen heterocyclischen Ring bilden:
X eine Bindung, O, CO, OCH₂, CH₂O oder SO₂ darstellt;
Z CO, CONR¹⁰ oder SO₂ darstellt;
R¹⁰ Wasserstoff, C₁₋₆-Alkyl, -C₃₋₈-Cycloalkyl, Aryl oder Heteroaryl darstellt;
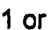 eine Einfach- oder eine Doppelbindung darstellt;
m und n unabhängig voneinander 0, 1 oder 2 darstellen;
R² Wasserstoff, C₁₋₆-Alkyl oder C₁₋₆-Alkoxy darstellt;
R³ Halogen, C₁₋₆-Alkyl, Hydroxy, C₁₋₆-Alkoxy, Cyano, Amino, -COC₁₋₆-Alkyl, -SO₂C₁₋₆-Alkyl oder Trifluormethyl darstellt;
R⁴ -(CH₂)_{q}-NR¹¹R¹² oder eine Gruppe der Formel (i) darstellt: 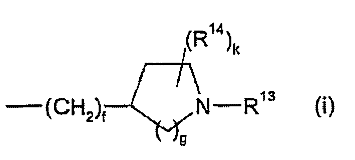 worin q 3 oder 4 darstellt;
-NR¹¹R¹² eine heterocyclische Gruppe darstellt, gegebenenfalls mit einer oder mehreren R¹⁷-Gruppen substituiert;
R¹³ C₁₋₆-Alkyl, C₃₋₈-Cycloalkyl, -C₁₋₆-Alkyl-C₁₋₆-alkoxy, -C₁₋₆-Alkyl-C₃₋₈-cycloalkyl darstellt;
R¹⁴ und R¹⁷ unabhängig voneinander Halogen, C₁₋₆-Alkyl, Halogenalkyl, OH oder C₁₋₆-Alkoxy darstellen;
f 0 oder 1 ist;
g 1 oder 2 ist;
k 0, 1 oder 2 ist;
oder ein pharmazeutisch annehmbares Salz davon.

2. Verbindung gemäß Anspruch 1, worin R¹ Folgendes darstellt:
- Aryl, gegebenenfalls mit 1 oder 2 Halogen-, Halogen-C₁₋₆-alkyl-, Cyano- oder SO₂Me-Gruppen substituiert;
- Aryl-X-heterocyclyl;
- Heteroaryl, gegebenenfalls mit 1 oder 2 Halogen-C₁₋₆-alkyl- oder Cyanogruppen substituiert;
- Heterocyclyl, gegebenenfalls mit 1 oder 2 Oxo-Gruppen substituiert; oder
- C₁₋₆-Alkyl-O-C₁₋₆-alkyl.

3. Verbindung gemäß Anspruch 2, worin R¹ Tetrahydropyranyl, 4-Cyanophenyl, 2-Cyanopyridin-3-yl oder 2-Trifluormethylpyridin-3-yl darstellt.

4. Verbindung gemäß Anspruch 3, worin R¹ 4-Cyanophenyl darstellt.

5. Verbindung gemäß einem der Ansprüche 1 bis 4, worin X und Z beide CO darstellen.

6. Verbindung gemäß einem der Ansprüche 1 bis 5, worin eine Einfachbindung darstellt.

7. Verbindung gemäß einem der Ansprüche 1 bis 6, worin m und n beide 0 darstellen.

8. Verbindung gemäß einem der Ansprüche 1 bis 7, worin R⁴ -(CH₂)q-NR¹¹R¹² darstellt, q 3 darstellt und -NR¹¹R¹² N-Piperidinyl oder N-Pyrrolidinyl darstellt, gegebenenfalls mit 1 oder 2 C₁₋₆-Alkylgruppen substituiert, oder R⁴ eine Gruppe der Formel (i) darstellt, worin f und k beide 0 darstellen, g 2 darstellt und R¹³ C₁₋₆-Alkyl oder C₃₋₈-Cycloalkyl darstellt.

9. Verbindung gemäß Anspruch 8, worin R⁴ eine Gruppe der Formel (i) darstellt, worin f und k beide 0 darstellen, g 2 darstellt und R¹³ i-Propyl darstellt.

10. Verbindung, die Folgendes ist:
4-(4-{[3-(1-Piperidinyl)propyl]oxy}phenyl)-1-(tetrahydro-2H-pyran-4-ylcarbonyl)piperidin; 4-{[4-(4-{[3-(1-Piperidinyl)propyl]oxy}phenyl)-1-piperidinyl]carbonyl}benzonitril; 4-{[4-(4{[3-(1-Piperidinyl)propyl]oxy}phenyl)-1-piperidinyl]carbonyl}pyridin; 4-(4-{[3-(1-Piperidinyl)propyl]oxy}phenyl)-1-{[4-(1-pyrrolidinylcarbonyl)phenyl]carbonyl}piperidin; 1-{[4-(Methylsulfonyl)phenyl]carbonyl}-4-(4-{[3-(1-piperidinyl)propyl]oxy}phenyl)piperidin; 1-[(4-Fluorphenyl)carbonyl]-4-(4-{[3-(1-piperidinyl)propyl]oxy}phenyl)piperidin; 3-{[4-(4-{[3-(1-Piperidinyl)propyl]oxy}phenyl)-1-piperidinyl]carbonyl}pyridin; 4-{[4-(4-{[3-(1-Piperidinyl)propyl]oxy}phenyl)-1-piperidinyl]carbonyl}morpholin; 1-(1-Piperidinylcarbonyl)-4-(4-{[3-(1-piperidinyl)propyl]oxy}phenyl)piperidin; 4-(4-{[3-(1-Piperidinyl)propyl]oxy}phenyl)-1-(1-pyrrolidinylcarbonyl)piperidin; 1-(4-Fluor-phenyl)-1-{4-[4-(1-isopropyl-piperidin-4-yloxy)phenyl]piperidin-1-yl}methanon; 1-(1-Methylethyl)-4-{[4-(1-{[4-(1-pyrrolidinylcarbonyl)phenyl]carbonyl}-4-piperidinyl)phenyl]oxy}piperidin; 1-(1-Methylethyl-4-({4-[1-(tetrahydro-2H-pyran-4-ylcarbonyl)-4-piperidinyl]phenyl}oxy)piperidin; 1-(1-Methylethyl)-4-{[4-(1-{[4-(methylsulfonyl)phenyl]carbonyl}-4-piperidinyl)phenyl]oxy}piperidin; 1-(1-(Methylethyl)-4-[(4-{1-[3-(methyloxy)propanoyl]-4-piperidinyl}phenyl)oxy]piperidin; 4-{[4-(4-{[1-(1-Methylethyl)-4-piperidinyl]oxy}phenyl)-1-piperidinyl]carbonyl}pyridin; 3-{[4-(4-{[1-(1-Methylethyl)-4-piperidinyl]oxy}phenyl)-1-piperidinyl]carbonyl}pyridin; 4-{[4-(4-{[1-(1-Methylethyl)-4-piperidinyl]oxy}phenyl)-1-piperidinyl]carbonyl}morpholin; 1-(1-Azetidinylcarbonyl)-4-(4-{[1-(1-methylethyl)-4-piperidinyl]oxy}phenyl]piperidin; 1-(1-Methylethyl)-4-({4-[1-(1-pyrrolidinylcarbonyl)-4-piperidinyl]phenyl}oxy)piperidin; 1-(1-Methylethyl)-4-({4-[1-(1-piperidinylcarbonyl)-4-piperidinyl]phenyl}oxy)piperidin; 4-{[4-(4-{[1-(1-Methylethyl)-4-piperidinyl]oxy}phenyl)-1-piperidinyl]carbonyl}thiomorpholin-1,1-dioxid; 4-[(4-{4-[(1-Cyclobutyl-4-piperidinyl)oxy]phenyl}-1-piperidinyl)carbonyl]benzonitril; 1-Cyclobutyl-4-[(4-{1-[(4-fluorphenyl)carbonyl]-4-piperidinyl}phenyl)oxy]piperidin; 1-Cyclobutyl-4-{[4-(1-{[4-(1-pyrrolidinylcarbonyl)phenyl]carbonyl}-4-piperidinyl)phenyl]oxy}piperidin; 1-Cyclobutyl-4-[(4-{1-[3-(methyloxy)propanoyl]-4-piperidinyl}phenyl)oxy]piperidin; 4-[(4-{4-[(1-Cyclobutyl-4-piperidinyl)oxy]phenyl}-1-piperidinyl)carbonyl]pyridin; 3-[(4-{4-[(1-Cyclobutyl-4-piperidinyl)oxy]phenyl}-1-piperidinyl)carbonyl]pyridin; 4-[(4-{4-[(1-Cyclobutyl-4-piperidinyl)oxy]phenyl}-1-piperidinyl)carbonyl]morpholin; 1-[(4-Fluorphenyl)carbonyl]-4-(4-{[3-(1-piperidinyl)propyl]oxy}phenyl)-1,2,3,6-tetrahydropyridin; 4-{[4-(4-{[3-(1-Piperidinyl)propyl]oxy}phenyl)-3,6-dihydro-1(2H)-pyridinyl]carbonyl}benzonitril; 4-(4-{[3-(1-Piperidinyl)propyl]oxy}phenyl)-1-{[4-(1-pyrrolidinylcarbonyl)phenyl]carbonyl}-1,2,3,6-tetrahydropyridin; 4-(4-{[3-(1-Piperidinyl)propyl]oxy}phenyl)-1-(tetrahydro-2H-pyran-4-ylcarbonyl)-1,2,3,6-tetrahydropyridin; 1-{[4-(Methylsulfonyl)phenyl]carbonyl}-4-(4-{[3-(1-piperidinyl)propyl]oxy}phenyl)-1,2,3,6-tetrahydropyridin; 4-{[4-(4-{[3-(1-Piperidinyl)propyl]oxy}phenyl)-3,6-dihydro-1(2H)-pyridinyl]carbonyl}morpholin; 1-(1-Piperidinylcarbonyl)-4-(4-{[3-(1-piperidinyl)propyl]oxy}phenyl)-1,2,3,6-tetrahydropyridin; 4-(4-{[3-(1-Piperidinyl)propyl]oxy}phenyl)-1-(1-pyrrolidinylcarbonyl)-1,2,3,6-tetrahydropyridin; 1-[(4-Fluorphenyl)carbonyl]-4-(4-{[1-(1-methylethyl)-4-piperidinyl]oxy}phenyl)-1,2,3,6-tetrahydropyridin; 4-{[4-(4-{[1-(1-Methylethyl)-4-piperidinyl]oxy}phenyl)-3,6-dihydro-1(2H)-pyridinyl]carbonyl}benzonitril; 4-(4-{[1-(1-Methylethyl)-4-piperidinyl]oxy}phenyl)-1-{[4-(1-pyrrolidinylcarbonyl)phenyl]carbonyl}-1,2,3,6-tetrahydropyridin; 4-(4-{[1-(1-Methylethyl)-4-piperidinyl]oxy}phenyl)-1-(tetrahydro-2H-pyran-4-ylcarbonyl)-1,2,3,6-tetrahydropyridin; 4-(4-{[1-(1-Methylethyl)-4-piperidinyl]oxy}phenyl)-1-{[4-(methylsulfonyl)phenyl]carbonyl}-1,2,3,6-tetrahydropyridin; 4-{[4-(4-{[1-(1-Methylethyl)-4-piperidinyl]oxy}phenyl)-3,6-dihydro-1(2H)-pyridinyl]carbonyl}pyridin; 4-{[4-(4-{[1-(1-Methylethyl)-4-piperidinyl]oxy}phenyl)-3,6-dihydro-1(2H)-pyridinyl]carbonyl}morpholin; 4-(4-{[1-(1-Methylethyl)-4-piperidinyl]oxy}phenyl)-1-(1-piperidinylcarbonyl)-1,2,3,6-tetrahydropyridin; 4-(4-{[1-(1-Methylethyl)-4-piperidinyl]oxy}phenyl)-1-(1-pyrrolidinylcarbonyl)-1,2,3,6-tetrahydropyridin; 4-({4-[4-({3-[(2R)-2-Methyl-1-pyrrolidinyl]propyl}oxy)phenyl]-1-piperidinyl}carbonyl)benzonitril; 4-[4-({3-[(2R)-2-Methyl-1-pyrrolidinyl]propyl}oxy)phenyl]-1-(tetrahydro-2H-pyran-4-ylcarbonyl)piperidin; 4-[4-({3-[(2R,5R)-2,5-Dimethyl-1-pyrrolidinyl]propyl}oxy)phenyl]-1-(tetrahydro-2H-pyran-4-ylcarbonyl)piperidin; 2-{[4-(4-{[1-(1-Methylethyl)-4-piperidinyl]oxy}phenyl)-1-piperidinyl]carbonyl}pyrazin; 3-{[4-(4-{[1-(1-Methylethyl)-4-piperidinyl]oxy}phenyl)-1-piperidinyl]carbonyl}benzonitril; 1-(1-Methylethyl)-4-{[4-(1-{[4-trifluormethyl)phenyl]carbonyl}-4-piperidinyl)phenyl]oxy}piperidin; 6-{[4-(4-{[1-(1-Methylethyl)-4-piperidinyl]oxy}phenyl)-1-piperidinyl]carbonyl}chinoxalin; oder ein pharmazeutisch annehmbares Salz davon.

11. Verbindung, die Folgendes ist:
5-{[4-(4-{[1-(1-Methylethyl)-4-piperidinyl]oxy}phenyl)-1-piperidinyl]carbonyl}-2-pyridincarbonitril; oder 5-{[4-(4-{[1-(1-Methylethyl)-4-piperidinyl]oxy}phenyl)-1-piperidinyl]carbonyl}-2-(trifluormethyl)pyridin; oder ein pharmazeutisch annehmbares Salz davon.

12. Verbindung, die Folgendes ist:
4-{[4-(4-{[1-(1-Methylethyl)-4-piperidinyl]oxy}phenyl)-1-piperidinyl]carbonyl}benzonitril oder ein pharmazeutisch annehmbares Salz davon.

13. Verbindung, die 1-(1-Methylethyl)-4-{[4-(1-{[1-pyrrolidinylcarbonyl)phenyl]carbonyl}-4-piperidiniyl)phenyl]oxy}piperidin ist, oder ein pharmazeutisch annehmbares Salz davon.

14. Pharmazeutische Zusammensetzung, die eine Verbindung der Formel (I) gemäß einem der Ansprüche 1 bis 13 oder ein pharmazeutisch annehmbares Salz davon und einen pharmazeutisch annehmbaren Träger oder Exzipienten umfasst.

15. Verbindung gemäß einem der Ansprüche 1 bis 13 oder ein pharmazeutisch annehmbares Salz davon zur Verwendung in der Therapie.

16. Verbindung gemäß einem der Ansprüche 1 bis 13 oder ein pharmazeutisch annehmbares Salz davon zur Verwendung in der Behandlung von neurologischen Krankheiten.

17. Verbindung gemäß einem der Ansprüche 1 bis 13 oder ein pharmazeutisch annehmbares Salz davon zur Verwendung in der Behandlung von Fettleibigkeit, Asthma, allergischer Rhinitis, nasaler Verstopfung, chronisch-obstruktiver Lungenerkrankung und gastrointestinalen Störungen.

18. Verwendung einer Verbindung gemäß einem der Ansprüche 1 bis 13 oder eines pharmazeutisch annehmbaren Salzes davon in der Herstellung eines Medikaments zur Behandlung von neurologischen Erkrankungen.

19. Verwendung einer Verbindung gemäß einem der Ansprüche 1 bis 13 oder eines pharmazeutisch annehmbaren Salzes davon in der Herstellung eines Medikaments zur Behandlung von Fettleibigkeit, Asthma, allergischer Rhinitis, nasaler Verstopfung, chronisch-obstruktiver Lungenerkrankung und gastrointestinalen Störungen.

20. Verfahren zur Herstellung einer Verbindung der Formel (I) oder eines pharmazeutisch annehmbaren Salzes davon, das folgende Schritte umfasst:
(a) Herstellen einer Verbindung der Formel (I), worin Z CO darstellt, das das Umsetzen einer Verbindung der Formel (II) 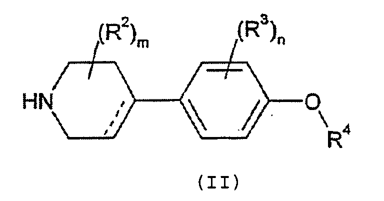 oder eines gegebenenfalls aktivierten oder geschützten Derivats davon,
worin 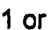 , R², R³, R⁴, m und n wie in Anspruch 1 definiert sind, mit einer Verbindung der Formel R¹-CO-L¹ umfasst, worin R¹ wie in Anspruch 1 definiert ist und L¹ eine geeignete Abgangsgruppe darstellt, wie z. B. ein geeignetes Halogenatom, oder eine Hydroxylgruppe; oder
(b) Herstellen einer Verbindung der Formel (I), worin Z SO₂ darstellt, das das Umsetzen einer Verbindung der Formel (II) mit einer Verbindung der Formel R¹-SO₂-L² umfasst, worin R¹ wie in Anspruch 1 definiert ist und L² eine geeignete Abgangsgruppe darstellt, wie z. B. ein geeignetes Halogenatom (z. B. Chlor); oder
(c) Herstellen einer Verbindung der Formel (I), worin Z CONH darstellt, das das Umsetzen einer Verbindung der Formel (II) mit einer Verbindung der Formel R¹-N=C=O umfasst, worin R¹ wie in Anspruch 1 definiert ist; oder
(d) Herstellen einer Verbindung der Formel (I), worin Z CONR¹⁰ darstellt, das das Umsetzen einer Verbindung der Formel (II) mit einer Verbindung der Formel R¹R¹⁰N-L³ umfasst, worin R¹ und R¹⁰ wie in Anspruch 1 definiert sind und L³ Wasserstoff oder COCl darstellt; oder
(e) Entschützen einer Verbindung der Formel (I) oder Umwandeln von Gruppen, die geschützte sind; und gegebenenfalls danach
(f) gegenseitiges Umwandeln in andere Verbindungen der Formel (I).

## Revendications

1. Composé de formule (I) : 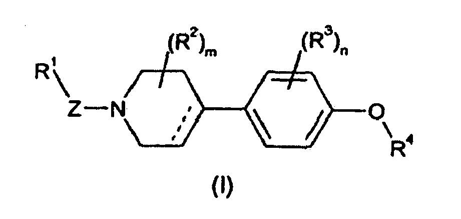 dans laquelle :
R¹ représente un groupe -alkyl(en C₁₋₆)-O-alkyle en C₁₋₆, -cycloalkyle en C₃₋₈, aryle, hétérocyclyle, hétéroaryle, -alkyl(en C₁₋₆)-aryle, -alkyl(en C₁₋₆)-hétéroaryle, -alkyl(en C₁₋₆)-hétérocyclyle, -aryl-X-aryle, -aryl-X-hétéroaryle, -aryl-X-hétérocyclyle, -hétéroaryl-X-aryle, -hétéroaryl-X-hétéroaryle, -hétéroaryl-X-hétérocyclyle, -hétérocyclyl-X-aryle, -hétérocyclyl-X-hétéroaryle ou -hétérocyclyl-X-hétérocyclyle,
dans laquelle lesdits groupes alkyle en C₁₋₆, cycloalkyle en C₃₋₈, aryle, hétéroaryle et hétérocyclyle de R¹ peuvent être éventuellement substitués par un ou plusieurs (par exemple 1, 2 ou 3) substituants qui peuvent être identiques ou différents, et qui sont choisis dans le groupe consistant en atomes d'halogène, groupes hydroxy, cyano, nitro, oxo, haloalkyle en C₁₋₆, polyhaloalkyle en C₁₋₆, haloalcoxy en C₁₋₆, polyhaloalcoxy en C₁₋₆, alkyle en C₁₋₆, alcoxy en C₁₋₆, alkyl(en C₁₋₆)thio, alcoxy(en C₁₋₆)alkyle en C₁₋₆, cycloalkyl(en C₃₋₇)alcoxy en C₁₋₆, alcanoyle en C₁₋₆, alcoxy(en C₁₋₆)carbonyle, alkyl(en C₁₋₆)sulfonyle, alkyl(en C₁₋₆)sulfinyle, alkyl(en C₁₋₆)sulfonyloxy, alkyl(en C₁₋₆)sulfonylalkyle en C₁₋₆, alkyl(en C₁₋₆)sulfonamidoalkyle en C₁₋₆, alkyl(en C₁₋₆)amidoalkyle en C₁₋₆, arylsulfonyle, arylsulfonyloxy, aryloxy, arylsulfonamido, arylcarboxamido, aroyle, ou un groupe -NR¹⁵R¹⁶, -CONR¹⁵R¹⁶, -NR¹⁵COR¹⁶, -NR¹⁵SO₂R¹⁶ ou -SO₂NR¹⁵R¹⁶, dans lequel R¹⁵ et R¹⁶ représentent indépendamment un atome d'hydrogène ou un groupe alkyle en C₁₋₆ ou forment ensemble un noyau hétérocyclique ;
X représente une liaison, O, CO, OCH₂, CH₂O ou SO₂;
Z représente CO, CONR¹⁰ ou SO₂;
R¹⁰ représente un atome d'hydrogène, un groupe alkyle en C₁₋₆, -cycloalkyle en C₃₋₈, aryle, hétérocyclyle, hétéroaryle;
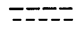 une simple liaison ou une double liaison;
m et n représentent indépendamment 0,1 ou 2;
R² représente un atome d'hydrogène, un groupe alkyle en C₁₋₆ ou alcoxy en C₁₋₆ ;
R³ représente un atome d'halogène, un groupe alkyle en C₁₋₆, hydroxy, alcoxy en C₁₋₆, cyano, amino, -COCalkyle en C₁₋₆, -SO₂alkyle en C₁₋₆ ou trifluorométhyle ;
R⁴ représente -(CH₂)_{q}-NR¹¹R¹² ou un groupe de formule (i): 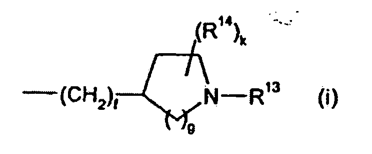 dans laquelle q représente 3 ou 4 ;
-NR¹¹R¹² représente un groupe hétérocyclique, éventuellement substitué par un ou plusieurs groupes R¹⁷ ;
R¹³ représente un groupe alkyle en C₁₋₆, -cycloalkyle en C₃₋₈, alkyl(en C₁₋₆)alcoxy en C₁₋₆, alkyl(en C₁₋₆)Cycloalkyle en C₃₋₈;
R¹⁴ et R¹⁷ représentent indépendamment un atome d'halogène, un groupe alkyle en C₁₋₆, haloalkyle, OH ou alcoxy en C₁₋₆ ;
f est 0 ou 1;
g est 1 ou 2;
k est 0,1 ou 2;
ou un sel de celui-ci acceptable du point de vue pharmaceutique.

2. Composé selon la revendication 1, dans laquelle R¹ représente :
un groupe aryle éventuellement substitué par 1 ou 2 atomes d'halogène, groupes haloalkyle en C₁₋₆, cyano ou SO₂Me ;
un groupe aryl-X-hétérocyclyle ;
un groupe hétéroaryle éventuellement substitué par 1 ou 2 groupes haloalkyle en C₁₋₆ ou cyano ;
un groupe hétérocyclyle éventuellement substitué par 1 ou 2 groupes oxo ; ou bien
un groupe alkyl(en C₁₋₆)-O-alkyle en C₁₋₆.

3. Composé selon la revendication 2, dans lequel R¹ représente un groupe tétrahydropyranyle, 4-cyanophényle, 2-cyanopyridin-3-yle ou 2-trifluorométhylpyridin-3-yle.

4. Composé selon la revendication 3, dans lequel R¹ représente un groupe 4-cyanophényle.

5. Composé selon l'une quelconque des revendications 1 à 4, dans lequel X et Z représentent tous les deux un groupe CO.

6. Composé selon l'une quelconque des revendications 1 à 5, dans lequel 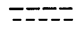 représente une simple liaison.

7. Composé selon l'une quelconque des revendications 1 à 6, dans lequel m et n représentent tous les deux 0.

8. Composé selon l'une quelconque des revendications 1 à 7, dans lequel R⁴ représente -(CH₂)_{q}-NR¹¹R¹², q représente 3 et -NR¹¹R¹² représente un groupe N-pipéridinyle ou N-pyrrolidinyle éventuellement substitué par 1 ou 2 groupes alkyles en C₁₋₆ ou bien R⁴ représente un groupe de formule (i) dans laquelle f et k représentent tous les deux 0, g représente 2 et R¹³ représente un groupe alkyle en C₁₋₆ ou cycloalkyle en C₃₋₈.

9. Composé selon la revendication 8,dans lequel R⁴ représente un groupe de formule (i) dans laquelle f et k représentent tous les deux 0, g représente 2 et R ¹³ représente un groupe isopropyle.

10. Composé qui est:
la 4-(4-{[3-(1-pipéridinyl)propyl]oxy}phényl)-1-(tétrahydro-2H-pyran-4-ylcarbonyl)pipéridine ;
le 4-{[4-(4-{[3-(1-pipéridinyl)propyl]oxy}phényl)-1-pipéridinyl]-carbonyl}benzonitrile ;
la 4-{[4-(4-{[3-(1-pipéridinyl)propyl]oxy}phényl)-1-pipéridinyl]-carbonyl}pyridine ;
la 4-(4-{[3-(1-pipéridinyl)propyl]oxy}phényl)-1-{[4-(1-pyrrolidinyl-carbonyl)phényl]carbonyl}pipéridine ;
la 1-{[4-(méthylsulfonyl)phényl]carbonyl}-4-(4-{[3-(1-pipéridinyl)-propyl]oxy}phényl)pipéridine ;
la 1-[(4-fluorophényl)carbonyl]-4-(4-{[3-(1-pipéridinyl)propyl]-oxy}phényl)pipéridine ;
la 3-{[4-(4-{[3-(1-pipéridinyl)propyl]oxy}phényl)-1-pipéridinyl]-carbonyl}pyridine ;
la 4- {[4-(4- ([3-(1 -pipéridinyl)propyl]oxy)phényl)- 1 -pipéridinyl]-carbonyl}morpholine ;
la 1-(1-pipéridinylcarbonyl)-4-(4-{[3-(1-pipéridinyl)propyl]oxy}-phényl)pipéridine ;
la 4-(4-{[3-(1-pipéridinyl)propyl]oxy}phényl)-1-(1-pyrrolidinyl-carbonyl)pipéridine ;
la 1-(4-fluorophényl)-1-{4-[4-(1-isopropyl-pipéridin-4-yloxy)-phényl]-pipéridin-1-yl}-méthanone ;
la 1-(1-méthyléthyl)-4-{[4-(1-{[4-(1-pyrrolidinylcarbonyl)-phényl]-carbonyl}-4-pipéridinyl)phényl]oxy}pipéridine ;
la 1-(1-méthyléthyl)-4-({4-[1-(tétrahydro-2H-pyran-4-ylcarbonyl)-4-pipéridinyl]phényl}oxy)pipéridine ;
la 1-(1-méthyléthyl)-4-{[4-(1-{[4-(méthylsulfonyl)phényl]-carbonyl}-4-pipéridinyl)phényl]oxy}-pipéridine ;
la 1-(1-méthyléthyl)-4-[(4-{1-(3-(méthyloxy)propanoyl]-4-pipéridinyl}phényl)oxy]pipéridine ;
la 4-{[4-(4-{[1-(1-méthyléthyl)-4-pipéridinyl]oxy}phényl)-1-pipéridinyl]carbonyl}pyridine ;
la 3-{[4-(4-{[1-(1-méthyléthyl)-4-pipéridinyl]oxy}phényl)-1-pipéridinyl]carbonyl}pyridine ;
la 4-{[4-(4-{[1-(1-méthyléthyl)-4-pipéridinyl]oxy}phényl)-1-pipéridinyl)carbonyl}morpholine ;
la 1-(1-azétidinylcarbonyl)-4-(4-{[1-(1-méthyléthyl)-4-pipéridinyl]-oxy}phényl)pipéridine ;
la 1-(1-méthyléthyl)-4-({4-[1-(1-pyrrolidinylcarbonyl)-4-pipéridinyl]phényl}oxy)pipéridine ;
la 1-(1-méthyléthyl)-4-({4-[1-(1-pipéridinylcarbonyl)-4-pipéridinyl]phényl}oxy)pipéridine;
le 4-{[4-(4-{[1-(1-méthyléthyl)-4-pipéridinyl]oxy}phényl)-1-pipéridinyl]carbonyl}thiomorpholine 1,1-dioxyde ;
le 4-[(4-{4-[(1-cyclobutyl-4-pipéridinyl)oxy]phényl}-1-pipéridinyl)-carbonyl]benzonitrile ;
la 1-cyclobutyl-4-[(4-{1-[(4-fluorophényl)carbonyl]-4-pipéridinyl}-phényl)oxy]pipéridine ;
la 1-cyclobutyl-4-{[4-(1-{[4-(1-pyrrolidinylcarbonyl)-phényl]-carbonyl}-4-pipéridinyl)phényl]oxy}pipéridine ;
la 1-cyclobutyl-4-[(4-{1-[3-(méthyloxy)propanoyl]-4-pipéridinyl}-phényl)oxy]pipéridine ;
la 4-[(4-{4-[(1-cyclobutyl-4-pipéridinyl)oxy]phényl}-1-pipéridinyl)carbonyl]pyridine ;
la 3-[(4-{4-[(1-cyclobutyl-4-pipéridinyl)oxy]phényl}-1-pipéridinyl)carbonyl]pyridine ;
la 4-[(4-{4-[(1-cyclobutyl-4-pipéridinyl)oxy]phényl}-1-pipéridinyl)carbonyl]morpholine ;
la 1-[(4-fluorophényl)carbonyl]-4-(4-{[3-(1-pipéridinyl)-propyl]-oxy}phényl)-1,2,3,6-tétrahydropyridine ;
le 4-{[4-(4-{[3-(1-pipéridinyl)propyl]oxy}phényl)-3,6-dihydro-1(2H)-pyridinyl]carbonyl}benzonitrile ;
la 4-(4-{[3-(1-pipéridinyl)propyl]oxy}phényl)-1-{[4-(1-pyrrolidinylcarbonyl)phényl]carbonyl}-1,2,3,6-tétrahydropyridine ;
la 4-(4-{[3-(1-pipéridinyl)propyl]oxy}phényl)-1-(tétrahydro-2H-pyran-4-ylcarbonyl)-1,2,3,6-tétrahydropyridine ;
la 1-{[4-(méthylsulfonyl)phényl]carbonyl}-4-(4-{[3-(1-pipéridinyl)-propyl]oxy}phényl)-1,2,3,6-tétrahydropyridine ;
la 4-{[4-(4-{[3-(1-pipéridinyl)propyl]oxy}phényl)-3,6-dihydro-1(2H)-pyridinyl]carbonyl}morpholine ;
la 1-(1-pipéridinylcarbonyl)-4-(4-{[3-(1-pipéridinyl)propyl]-oxy}-phényl)-1,2,3,6-tétrahydropyridine ;
la 4-(4-{[3-(1-pipéridinyl)propyl]oxy}phényl)-1-(1-pyrrolidinyl-carbonyl)-1,2,3,6-tétrahydropyridine ;
la 1-[(4-fluorophényl)carbonyl]-4-(4-{[1-(1-méthyléthyl)-4-pipéridinyl]oxy}phényl)-1,2,3,6-tétrahydropyridine ;
le 4-{[4-(4-{[1-(1-méthyléthyl)-4-pipéridinyl]oxy}phényl)-3,6-dihydro-1(2H)-pyridinyl]carbonyl}benzonitrile ;
la 4-(4-{[1-(1-méthyléthyl)-4-pipéridinyl]oxy}phényl)-1-{[4-(1-pyrrolidinylcarbonyl)phényl]carbonyl}-1,2,3,6-tétrahydropyridine ;
la 4-(4-{[1-(1-méthyléthyl)-4-pipéridinyl]oxy}phényl)-1-(tétrahydro-2H-pyran-4-ylcarbonyl)-1,2,3,6-tétrahydropyridine ;
la 4-(4-{[1-(1-méthyléthyl)-4-pipéridinyl]oxy}phényl)-1-{[4-(méthylsulfonyl)phényl]carbonyl}-1,2,3,6-tétrahydropyridine ;
la 4-{[4-(4-{[1-(1-méthyléthyl)-4-pipéridinyl]oxy}phényl)-3,6-dihydro-1(2H)-pyridinyl]carbonyl}pyridine ;
la 4-{[4-(4-{[1-(1-méthyléthyl)-4-pipéridinyl]oxy}phényl)-3,6-dihydro-1(2H)-pyridinyl]carbonyl}morpholine ;
la 4-(4-{,[1-(1-méthyléthyl)-4-pipéridinyl]oxy}phényl)-1-(1-pipéridinylcarbonyl)-1,2,3,6-tétrahydropyridine ;
la 4-(4-{[1-(1-méthyléthyl)-4-pipéridinyl]oxy}phényl)-1-(1-pyrrolidinylcarbonyl)-1,2,3,6-tétrahydropyridine ;
le 4-({4-[4-({3-[(2R)-2-méthyl-1-pyrrolidinyl]propyl}oxy)-phényl]-1-pipéridinyl}carbonyl)benzonitrile ;
la 4-[4-({3-[(2R)-2-méthyl-1-pyrrolidinyl]propyl}oxy)phényl]-1-(tétrahydro-2H-pyran-4-ylcarbonyl)pipéridine ;
la 4-[4-({3-[(2R,SR)-2,5-diméthyl-1-pyrrolidinyl]propyl}oxy)-phényl]-1-(tétrahydro-2H-pyran-4-ylcarbonyl)pipéridine ;
la 2-{[4-(4-{[1-(1-méthyléthyl)-4-pipéridinyl]oxy}phényl)-1-pipéridinyl]carbonyl}pyrazine ;
le 3-{[4-(4-{[1-(1-méthyléthyl)-4-pipéridinyl]oxy}phényl)-1-pipéridinyl]carbonyl}benzonitrile ;
la 1-(1-méthyléthyl)-4-{[4-(1-{[4-(trifluoromethyl)phényl]-carbonyl}-4-pipéridinyl)phényl]oxy}pipéridine ;
la 6-{[4-(4-{[1-(1-méthylethyl)-4-pipéridinyl]oxy}phényl)-1-pipéridinyl]carbonyl}quinoxaline;
ou un sel de ceux-ci acceptable du point de vue pharmaceutique.

11. Composé qui est :
le 5-{[4-(4-{[1-(1-méthyléthyl)-4-pipéridinyl]oxy}phényl)-1-pipéridinyl]carbonyl}-2-pyridinecarbonitrile, ou
la 5-{[4-(4-{[1-(1-méthyléthyl)-4-pipéridinyl]oxy}phényl)-1-pipéridinyl]carbonyl}-2-(trifluorométhyl)pyridine ;
ou un sel de ceux-ci acceptable du point de vue pharmaceutique.

12. Composé qui est :
le 4-{[4-(4-{[1-(1-méthyléthyl)-4-pipéridinyl]oxy}phényl)-1-pipéridinyl]carbonyl}benzonitrile
ou un sel de celui-ci acceptable du point de vue pharmaceutique.

13. Composé qui est :
la 1-(1-méthyléthyl)-4-{ [4-(1-{[4-(1-pyrrolidinylcarbonyl)-phényl]carbonyl}-4-pipéridinyl)phényl]oxy}pipéridine
ou un sel de celle-ci acceptable du point de vue pharmaceutique.

14. Composition pharmaceutique qui comprend un composé de formule (I) selon l'une quelconque des revendications 1 à 13 ou un sel de celui-ci acceptable du point de vue pharmaceutique et un support ou excipient acceptable du point de vue pharmaceutique.

15. Composé selon l'une quelconque des revendications 1 à 13 ou sel de celui-ci acceptable du point de vue pharmaceutique utiles en thérapie.

16. Composé selon l'une quelconque des revendications 1 à 13 ou sel de celui-ci acceptable du point de vue pharmaceutique utiles dans le traitement de maladies neurologiques.

17. Composé selon l'une quelconque des revendications 1 à 13 ou sel de celui-ci acceptable du point de vue pharmaceutique utiles dans le traitement de l'obésité, de l'asthme, de la rhinite allergique, de la congestion nasale, du syndrome respiratoire obstructif chronique et des troubles gastro-intestinaux.

18. Utilisation d'un composé selon l'une quelconque des revendications 1 à 13 ou d'un sel de celui-ci acceptable du point de vue pharmaceutique dans la fabrication d'un médicament destiné au traitement de maladies neurologiques.

19. Utilisation d'un composé selon l'une quelconque des revendications 1 à 13 ou d'un sel de celui-ci acceptable du point de vue pharmaceutique dans la fabrication d'un médicament destiné au traitement de l'obésité, de l'asthme, de la rhinite allergique, de la congestion nasale, du syndrome respiratoire obstructif chronique et des troubles gastro-intestinaux.

20. Procédé de préparation d'un composé de formule (I) ou d'un sel de celui-ci acceptable du point de vue pharmaceutique, lequel procédé comprend :
(a) la préparation d'un composé de formule (I) dans laquelle Z représente CO, qui comprend la réaction d'un composé de formule (II) : 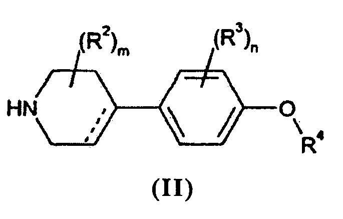 ou d'un dérivé de celui-ci éventuellement activé ou protégé, dans laquelle 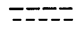 , R², R³, R⁴, m et n sont tels que définis selon la revendication 1, avec un composé de formule R¹-CO-L¹, dans laquelle R¹ est tel que défini selon la revendication 1 et L¹ représente un groupe mobile convenable tel qu'un atome d'halogène convenable, ou un groupe hydroxy ; ou bien
(b) la préparation d'un composé de formule (I) dans laquelle Z représente SO₂ qui comprend la réaction d'un composé de formule (II) avec un composé de formule R¹-SO₂-L², dans laquelle R¹ est tel que défini selon la revendication 1 et L² représente un groupe mobile convenable tel qu'un atome d'halogène convenable (par exemple un atome de chlore) ; ou bien
(c) la préparation d'un composé de formule (I) dans laquelle Z représente CONH, qui comprend la réaction d'un composé de formule (II) avec un composé de formule R¹-N=C=O, dans laquelle R¹ est tel que défini selon la revendication 1 ; ou bien
(d) la préparation d'un composé de formule (I) dans laquelle Z représente CONR¹⁰ qui comprend la réaction d'un composé de formule (II) avec un composé de formule R¹R¹⁰N-L³, dans laquelle R¹ et R¹⁰ sont tels que définis selon la revendication 1 et L³ représente un atome d'hydrogène ou un groupe COC1 ; ou bien
(e) la supression de la protection d'un composé de formule (I) ou la conversion des groupes qui sont protégés ; et éventuellement ensuite
(f) une interconversion en d'autres composés de formule (I).
